# EUROPEAN PATENT SPECIFICATION

(11) **EP 0 687 267 B1**
(45) Date of publication and mention of the grant of the patent: **01.09.1999**
(21) Application number: 94906978.5
(22) Date of filing: 25.02.1994
(51) Int. Cl.: C07D 471/04

(54) **PYRROLO-PYRIDINE DERIVATIVES AS LIGANDS OF DOPAMINE RECEPTOR**
PYRROLOPYRIDINDERIVATE ALS DOPAMINREZEPTOR LIGANDEN
DERIVES DE PYRROLO-PYRIDINE COMME LIGANDS DES RECEPTEURS DOPAMINE

(30) Priority: 01.03.1993 GB 9304110; 05.08.1993 GB 9316260
(43) Date of publication of application: 20.12.1995
(73) Proprietor: MERCK SHARP & DOHME LTD., Hoddesdon Hertfordshire EN11 9BU (GB)
(72) Inventor: BAKER, Raymond, Harlow Essex CM20 2QR (GB); CURTIS, Neil, Roy, Harlow Essex CM20 2QR (GB); KULAGOWSKI, Janusz, Jozef, Harlow Essex CM20 2QR (GB); LEESON, Paul, David, Harlow Essex CM20 2QR (GB); SMITH, Adrian, Leonard, Harlow Essex CM20 2QR (GB); RIDGILL, Mark, Peter, Harlow Essex CM20 2QR (GB)
(74) Representative: Thompson, John Dr.
(86) International application number: GB9400384
(87) International publication number: WO9420459

(56) References cited:
- GB-A- 2 044 254
- JOURNAL OF MEDICINAL CHEMISTRY vol. 35 , 1992 , WASHINGTON US pages 4020 - 4026 H. BÖTTCHER ET AL. 'Synthesis and dopaminergic activity of some 3-(1,2,3,6- tetrahydro-1-pyridylalkyl)indoles. A novel conformational model to explain structure-activity relationships'

## Description

This invention relates to a particular class of heteroaromatic compounds. More particularly, the invention is concerned with substituted pyrrolo[2,3-b]pyridine derivatives which are ligands for dopamine receptor subtypes within the body and are therefore of use in the treatment and/or prevention of disorders of the dopamine system, including schizophrenia, depression, nausea, Parkinson's disease, tardive dyskinesias and extrapyramidal side-effects associated with treatment by conventional neuroleptic agents, neuroleptic malignant syndrome, and disorders of hypothalamic-pituitary function such as hyperprolactinaemia and amenorrhoea.

Upper gastrointestinal tract motility is believed to be under the control of the dopamine system. The compounds according to the present invention may thus be of use in the prevention and/or treatment of gastrointestinal disorders, and the facilitation of gastric emptying.

Dependence-inducing agents such as cocaine and amphetamine have been shown to interact with the dopamine system. Compounds capable of counteracting this effect, including the compounds in accordance with the present invention, may accordingly be of value in the prevention or reduction of dependence on a dependence-inducing agent.

Dopamine is known to be a peripheral vasodilator; for example, it has been shown to exert a dilatory effect on the renal vascular bed. This implies that the compounds of the present invention may be beneficial in controlling vascular blood flow.

The localisation of dopamine receptor mRNA in rat heart and large vessels has been noted. This suggests a role for dopamine receptor ligands in controlling cardiovascular function, either by affecting cardiac and smooth muscle contractility or by modulating the secretion of vasoactive substances. The compounds according to the present invention may therefore be of assistance in the prevention and/or treatment of such conditions as hypertension and congestive heart failure.

Molecular biological techniques have revealed the existence of several subtypes of the dopamine receptor. The dopamine D1 receptor subtype has been shown to occur in at least two discrete forms. Two forms of the D2 receptor subtype, and at least one form of the D3 receptor subtype, have also been discovered. More recently, the D4 (Van Tol et al., Nature (London), 1991, **350**, 610) and D5 (Sunahara et al., Nature (London), 1991, **350**, 614) receptor subtypes have been described.

The disclosure of GB-A-2044254 generically encompasses inter alia a class of 3-[piperidin-1-ylalkyl]-1H-pyrrolo[2,3-b]pyridine derivatives substituted on the piperidine ring by an isoindoledione or like moiety. These compounds are alleged therein to be useful as antidepressants. There is, however, no specific disclosure in GB-A-2044254 of a substituted pyrrolo[2,3-b]pyridine derivative, nor indeed any suggestion that such compounds would be of benefit in the treatment and/or prevention of disorders of the dopamine system.

J. Med. Chem., 1992, **35**, 4020-4026, describes the activity of a family of indole analogues as dopamine receptor ligands.

The compounds in accordance with the present invention, being ligands for dopamine receptor subtypes within the body, are accordingly of use in the treatment and/or prevention of disorders of the dopamine system.

The present invention accordingly provides a compound of formula I, or a salt thereof: 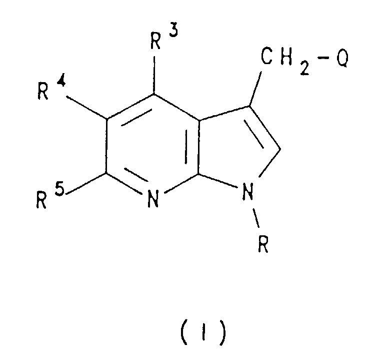 wherein
R represents hydrogen or C₁₋₆ alkyl;
Q represents a moiety of formula Qa, Qb or Qc: 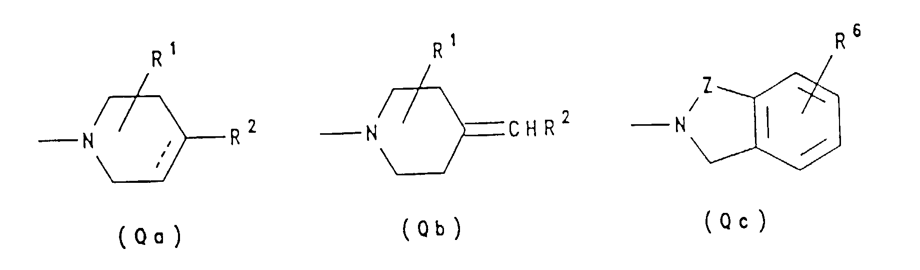 in which the broken line represents an optional chemical bond;
   R¹ represents hydrogen;
   R² represents C₁₋₆ alkyl, aryl, aryl(C₁₋₆)alkyl, aryloxy(C₁₋₆)alkyl, aryl(C₂₋₆)alkenyl, aryl(C₂₋₆)alkynyl, C₃₋₇ heterocycloalkyl(C₁₋₆)alkyl, heteroaryl, heteroaryl(C₁₋₆)alkyl or heteroaryl(C₂₋₆)alkenyl, any of which groups may be optionally substituted by one or more groups selected from C₁₋₆ alkyl, halogen, nitro, C₁₋₆ alkoxy, aryloxy and arylcarbonyloxy;
   R³, R⁴ and R⁵ independently represent hydrogen, halogen, cyano, trifluoromethyl, nitro, amino, C₁₋₆ alkylamino, di(C₁₋₆)alkylamino, C₁₋₆ alkyl, C₁₋₆ alkoxy, aryl(C₁₋₆)alkoxy or C₂₋₆ alkylcarbonyl;
   Z represents -CH₂- or -CH₂CH₂-; and
   R⁶ represents hydrogen, methoxy, phenyl, chlorophenyl, phenoxy, benzyloxy, thienyl, chloro or bromo.

For use in medicine, the salts of the compounds of formula I will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts.

Suitable alkyl groups include straight-chained and branched alkyl groups containing from 1 to 6 carbon atoms. Typical examples include methyl and ethyl groups, and straight-chained or branched propyl and butyl groups. Particular alkyl groups are methyl, ethyl, n-propyl, isopropyl and t-butyl.

Particular aryl groups include phenyl and naphthyl.

Particular aryl(C₁₋₆)alkyl groups include benzyl, naphthylmethyl, phenethyl and phenylpropyl.

A particular aryl(C₂₋₆)alkenyl group is phenylethenyl.

A particular aryl(C₂₋₆)alkynyl group is phenylethynyl.

A particular C₃₋₇ heterocycloalkyl(C₁₋₆)alkyl group is tetrahydrofurylethyl.

Suitable heteroaryl groups include pyridyl, quinolyl, isoquinolyl, pyridazinyl, pyrimidinyl, pyrazinyl, pyranyl, furyl, benzofuryl, dibenzofuryl, thienyl, benzthienyl, indolyl, indazolyl, imidazolyl, benzimidazolyl, oxadiazolyl and thiadiazolyl groups.

Particular heteroaryl(C₁₋₆)alkyl groups include thienylmethyl, pyridylmethyl, pyrimidinylmethyl, pyrazinylmethyl and furylethyl.

Particular heteroaryl(C₂₋₆)alkenyl groups include furylethenyl and thienylethenyl.

The term "halogen" as used herein includes fluorine, chlorine, bromine and iodine, especially chlorine.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof are encompassed within the scope of the present invention.

Suitably, the substituent R represents hydrogen or methyl, especially hydrogen.

Particular values of R² include methyl, benzoyloxy-methyl, ethyl, n-propyl, isopropyl, phenyl, chlorophenyl, ethylphenyl, methoxyphenyl, nitrophenyl, naphthyl, benzyl, chlorobenzyl, phenethyl, phenoxy-methyl, phenylethenyl, chloro-phenylethenyl, methoxy-phenylethenyl, phenylethynyl, tetrahydrofuryl-ethyl, indolyl, benzofuryl, benzthienyl, furylethyl, methyl-furylethyl, thienylethenyl and methyl-furylethenyl.

Particular values for the substituents R³, R⁴ and R⁵ include hydrogen, fluoro, chloro, methyl, methoxy and benzyloxy.

One sub-class of compounds according to the invention is represented by the compounds of formula IIA, and salts thereof: 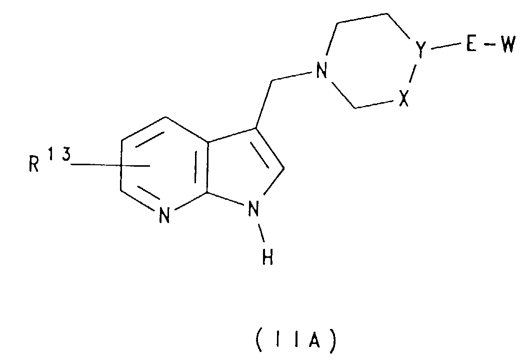 wherein
E represents -(CH₂)ₙ-, -CH=CH- or -C≡C-;
n is zero, 1, 2 or 3;
-X-Y- represents -CH₂-CH- or -CH=C-;
W represents a group of formula (i), (ii), (iii), (iv), (v) or (vi): 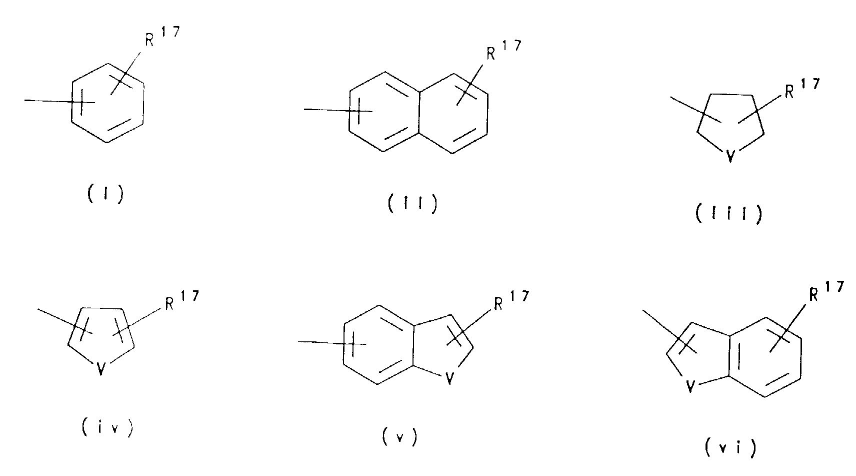 in which V represents oxygen, sulphur or NH;
   R¹³ represents hydrogen, halogen, cyano, nitro, trifluoromethyl, amino, C₁₋₆ alkylamino, di(C₁₋₆)alkylamino, C₁₋₆ alkyl, C₁₋₆ alkoxy, aryl(C₁₋₆)alkoxy or C₂₋₆ alkylcarbonyl; and
   R¹⁷ represents hydrogen, halogen, nitro, C₁₋₆ alkyl or C₁₋₆ alkoxy;
   provided that, when W represents a group of formula (iii), then E represents -(CH₂)ₙ- in which n is 1, 2 or 3; and also that, when W represents a group of formula (iv), (v) or (vi), then E does not represent -C≡C-.

Particular values of R¹³ include hydrogen, fluoro, chloro, methyl, ethyl, methoxy and benzyloxy.

Particular values of R¹⁷ include hydrogen, chloro, methyl, methoxy and nitro.

A particular subset of the compounds of formula IIA as defined above is represented by the compounds of formula IIB, and salts thereof: 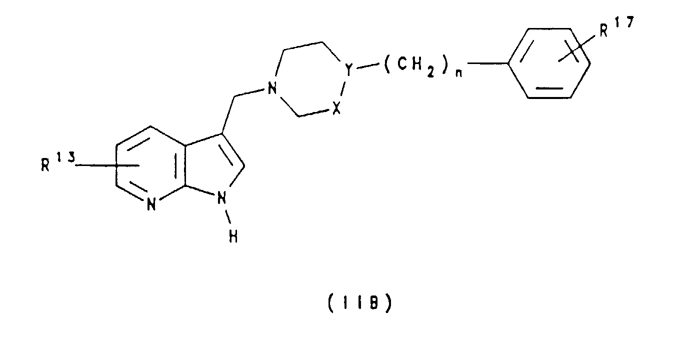 wherein n, X, Y, R¹³ and R¹⁷ are as defined with reference to formula IIA above.

Another sub-class of compounds according to the invention is represented by the compounds of formula IIC, and salts thereof: 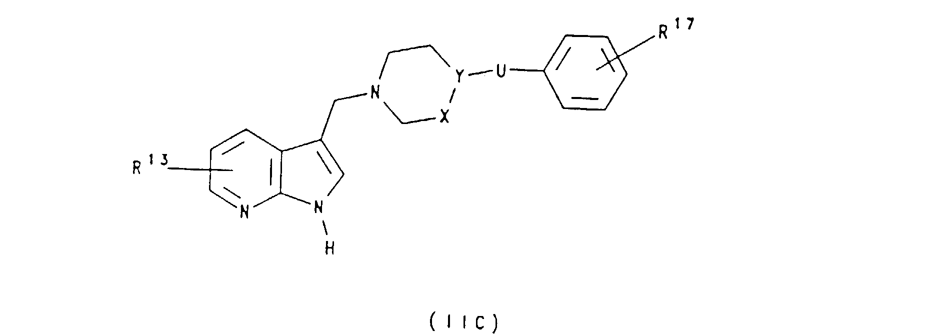 wherein
U represents -CH=CH-, -C≡C- or -CH₂O-; and
X, Y, R¹³ and R¹⁷ are as defined with reference to formula IIA above.

A particular subset of the compounds of formula IIC as defined above comprises those compounds wherein U represents -CH=CH-, in particular in the (E) configuration.

A further sub-class of compounds according to the invention is represented by the compounds of formula IID, and salts thereof: 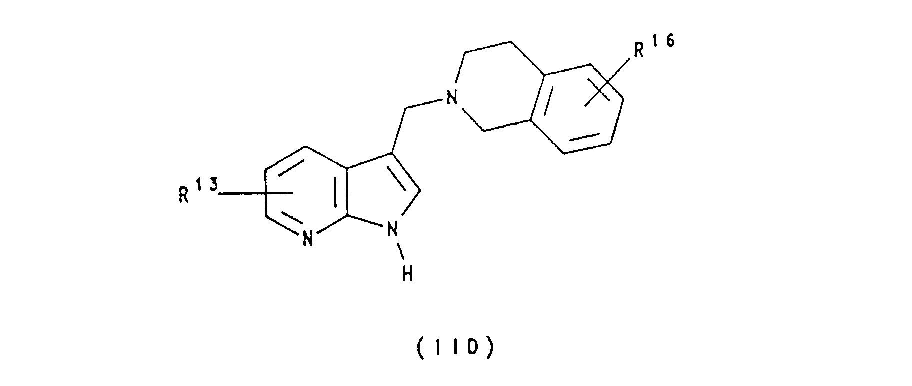 wherein
R¹³ is as defined with reference to formula IIA above; and
R¹⁶ represents hydrogen, methoxy, phenyl, chlorophenyl, phenoxy, benzyloxy, thienyl, chloro or bromo.

Specific compounds within the scope of the present invention include:
3-(1,2,3,4-tetrahydroisoquinolin-2-yl)methyl-1H-pyrrolo[2,3-b]pyridine;
3- [4-(2-phenylethyl)piperidin-1-yl]methyl-1H-pyrrolo[2, 3-b]pyridine;
3-(4-phenyl-1,2,3,6-tetrahydropyrid-1-yl)methyl-1H-pyrrolo[2,3-b]pyridine;
(E)-3-[4-(2-phenylethenyl)-1,2,3,6-tetrahydropyrid-1-yl]methyl-1H-pyrrolo[2,3-b]pyridine;
3-[4-(2-phenylethyl)-1,2,3,6-tetrahydropyrid-1-yl]methyl-1H-pyrrolo[2,3-b]pyridine;
3-[4-(naphth-2-yl)-1,2,3,6-tetrahydropyridin-1-yl]methyl-1H-pyrrolo[2,3-b]pyridine;
3-[4-(4-methoxyphenyl)-1,2,3,6-tetrahydropyridin-1-yl]methyl-1H-pyrrolo[2,3-b]pyridine;
3-[4-(1H-indol-5-yl)-1,2,3,6-tetrahydropyridin-1-yl]methyl-1H-pyrrolo[2,3-b]pyridine;
3-[4-(benzofuran-5-yl)-1,2,3,6-tetrahydropyridin-1-yl]methyl-1H-pyrrolo[2,3-b]pyridine;
3-[4-(benzofuran-6-yl)-1,2,3,6-tetrahydropyridin-1-yl]methyl-1H-pyrrolo[2,3-b]pyridine;
3-[4-(benzothiophen-2-yl)-1,2,3,6-tetrahydropyridin-1-yl]methyl-1H-pyrrolo[2,3-b]pyridine;
3-[4-(benzofuran-2-yl)-1,2,3,6-tetrahydropyridin-1-yl]methyl-1H-pyrrolo[2,3-b]pyridine;
3-[4-(2-phenylethynyl)-1,2,3,6-tetrahydropyridin-1-yl]methyl-1H-pyrrolo[2,3-b]pyridine;
(E)-3-[4-(2-(thiophen-3-yl)ethenyl)-1,2,3,6-tetrahydropyridin-1-yl]methyl-1H-pyrrolo[2,3-b]pyridine;
(E)-3-[4-(2-(2-chlorophenyl)ethenyl)-1,2,3,6-tetrahydropyridin-1-yl]methyl-1H-pyrrolo[2,3-b]pyridine;
(E)-3-[4-(2-(4-chlorophenyl)ethenyl)-1,2,3,6-tetrahydropyridin-1-yl]methyl-1H-pyrrolo[2,3-b]pyridine;
(E)-3-[4-(2-(thiophen-2-yl)ethenyl)-1,2,3,6-tetrahydropyridin-1-yl]methyl-1H-pyrrolo[2,3-b]pyridine;
3-[4-(2-(furan-2-yl)ethyl)-1,2,3,6-tetrahydropyridin-1-yl]methyl-1H-pyrrolo[2,3-b]pyridine;
3-[4-(2-(tetrahydrofuran-2-yl)ethyl)-1,2,3,6-tetrahydropyridin-1-yl]methyl-1H-pyrrolo[2,3-b]pyridine;
3-[4-(2-(5-methylfuran-2-yl)ethyl)-1,2,3,6-tetrahydropyridin-1-yl]methyl-1H-pyrrolo[2,3-b]pyridine;
3-(4-ethyl-1,2,3,6-tetrahydropyridin-1-yl)methyl-1H-pyrrolo[2,3-b]pyridine;
3-(4-benzoyloxymethyl-1,2,3,6-tetrahydropyridin-1-yl)methyl-1H-pyrrolo[2,3-b]pyridine;
(E)-3-[4-(2-(3-methoxyphenyl)ethenyl)-1,2,3,6-tetrahydropyridin-1-yl]methyl-1H-pyrrolo[2,3-b]pyridine;
3-(4-phenoxymethyl-1,2,3,6-tetrahydropyridin-1-yl)methyl-1H-pyrrolo[2,3-b]pyridine;
(E)-3-[4-(2-(5-methylfuran-2-yl)ethenyl)-1,2,3,6-tetrahydropyridin-1-yl]methyl-1H-pyrrolo[2,3-b]pyridine;
3-(6-phenyl-1,2,3,4-tetrahydroisoquinolin-2-yl)methyl-1H-pyrrolo[2,3-b]pyridine;
3-(6-methoxy-1,2,3,4-tetrahydroisoquinolin-2-yl)methyl-1H-pyrrolo[2,3-b]pyridine;
3-(7-phenyl-1,2,3,4-tetrahydroisoquinolin-2-yl)methyl-1H-pyrrolo[2,3-b]pyridine;
3-(7-benzyloxy-1,2,3,4-tetrahydroisoquinolin-2-yl)methyl-1H-pyrrolo[2,3-b]pyridine;
3-[7-(thiophen-3-yl)-1,2,3,4-tetrahydroisoquinolin-2-yl]methyl-1H-pyrrolo[2,3-b]pyridine;
3-[6-(4-chlorophenyl)-1,2,3,4-tetrahydroisoquinolin-2-yl]methyl-1H-pyrrolo[2,3-b]pyridine;
3-(5-phenyl-1,2,3,4-tetrahydroisoquinolin-2-yl)methyl-1H-pyrrolo[2,3-b]pyridine;
3-(5-benzyloxy-1,2,3,4-tetrahydroisoquinolin-2-yl)methyl-1H-pyrrolo[2,3-b]pyridine;
3-(5-phenoxy-1,2,3,4-tetrahydroisoquinolin-2-yl)methyl-1H-pyrrolo[2,3-b]pyridine;
and salts thereof.

The invention also provides pharmaceutical compositions comprising one or more compounds of this invention in association with a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, auto-injector devices or suppositories; for oral, parenteral, intranasal, sublingual or rectal administration, or for administration by inhalation or insufflation. Alternatively, the compositions may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

In the treatment of schizophrenia, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The compounds in accordance with the present invention may be prepared by a process which comprises reacting a compound of formula III with a compound of formula IV: 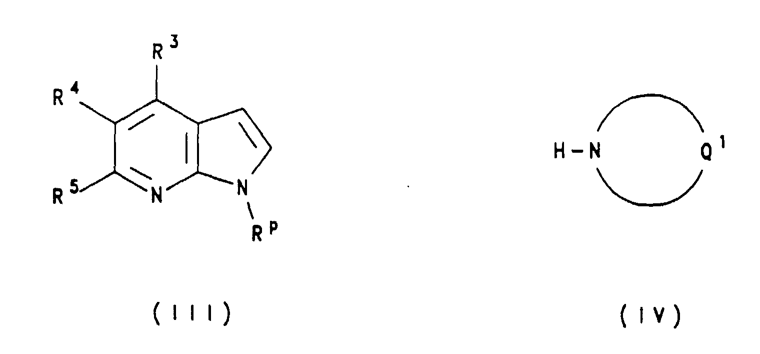 wherein R³, R⁴ and R⁵ are as defined above, Q¹ represents the residue of a moiety of formula Qa to Qc as defined above, and R^{p} corresponds to the group R as defined above or represents a suitable protecting group; in the presence of a substantially equimolar amount of formaldehyde; followed, where required, by removal of the protecting group R^{p}; and subsequently, if necessary, N-alkylation by standard methods to introduce the moiety R.

The reaction is conveniently carried out by stirring the reactants in aqueous acetic acid, ideally in the presence of a buffer such as sodium acetate trihydrate, suitably at room temperature.

The formaldehyde may be utilised in the form of paraformaldehyde; or as a solution of formaldehyde in an inert solvent, e.g. 37% aqueous formaldehyde.

The protecting group R^{p}, when present, is suitably an acyl moiety such as acetyl, which can conveniently be removed as necessary by treatment under strongly basic conditions, e.g. sodium methoxide in methanol. Alternatively, the protecting group R^{p} may be a carbamoyl moiety such as t-butoxycarbonyl (BOC), which can conveniently be removed as necessary by treatment under mildly acidic conditions.

In an alternative procedure, the compounds according to the present invention may be prepared by a process which comprises reacting a compound of formula IV as defined above with a compound of formula V: 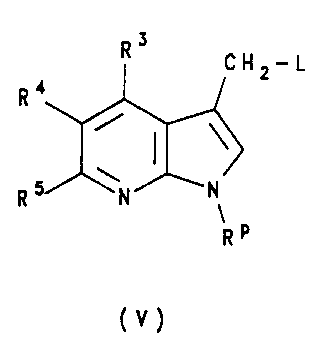 wherein R³, R⁴, R⁵ and R^{p} are as defined above, and L represents a suitable leaving group; followed, where required, by removal of the protecting group R^{p}; and subsequently, if necessary, N-alkylation by standard methods to introduce the moiety R.

The leaving group L is suitably a halogen atom, e.g. chlorine or bromine; or a dialkylamino group, e.g. dimethylamino.

When L represents a halogen atom, the reaction between compounds IV and V is conveniently carried out by stirring the reactants under basic conditions in a suitable solvent, for example potassium carbonate in N,N-dimethylformamide, or triethylamine in tetrahydrofuran or acetonitrile. Where L represents a dialkylamino group, the reaction is conveniently effected by heating the reactants in an inert solvent such as toluene, typically at the reflux temperature of the solvent.

Where they are not commercially available, the starting materials of formula III, IV and V may be prepared by procedures analogous to those described in the accompanying Examples, or by standard methods well known from the art.

It will be appreciated that any compound of formula I initially obtained from any of the above processes may, where appropriate, subsequently be elaborated into a further desired compound of formula I using techniques known from the art. For example, a compound of formula I wherein R is hydrogen initially obtained may be converted into a compound of formula I wherein R represents C₁₋₆ alkyl by standard alkylation techniques, such as by treatment with an alkyl iodide, e.g. methyl iodide, typically under basic conditions, e.g. sodium hydride in dimethylformamide, or triethylamine in acetonitrile.

Where the above-described processes for the preparation of the compounds according to the invention give rise to mixtures of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques such as preparative HPLC, or the formation of diastereomeric pairs by salt formation with an optically active acid, such as (-)-di-p-toluoyl-d-tartaric acid and/or (+)-di-p-toluoyl-l-tartaric acid, followed by fractional crystallization and regeneration of the free base. The compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The following Examples illustrate the preparation of compounds according to the invention.

The compounds useful in this invention potently inhibit [³H]-spiperone binding to human dopamine D₄ receptor subtypes expressed in clonal cell lines.

### [³H]-Spiperone Binding Studies

Clonal cell lines expressing the human dopamine D₄ receptor subtype were harvested in PBS and then lysed in 10 mM Tris-HCl pH 7.4 buffer containing 5 mM MgSO₄ for 20 min on ice. Membranes were centrifuged at 50,000g for 15 min at 4°C and the resulting pellets resuspended in assay buffer (50 mM Tris-HCl pH 7.4 containing 5 mM EDTA, 1.5 mM CaCl₂, 5 mM MgCl₂, 5 mM KCl, 120 mM NaCl, and 0.1% ascorbic acid) at 20 mg/ml wet weight. Incubations were carried out for 60 min at room temperature (22°C) in the presence of 0.05-2 nM [³H]-spiperone or 0.2 nM for displacement studies and were initiated by addition of 20-100 µg protein in a final assay volume of 0.5 ml. The incubation was terminated by rapid filtration over GF/B filters presoaked in 0.3% PEI and washed with 10 ml ice-cold 50 mM Tris-HCl, pH 7.4. Specific binding was determined by 10 µM apomorphine and radioactivity determined by counting in a LKB beta counter. Binding parameters were determined by non-linear least squares regression analysis, from which the inhibition constant Kᵢ could be calculated for each test compound.

The compounds of the accompanying Examples were tested in the above assay, and all were found to possess a Kᵢ value for displacement of [³H]-spiperone from the human dopamine D₄ receptor subtype of below 1.5 µM.

### EXAMPLE 1

### 3-(1,2,3,4-Tetrahydroisoquinolin-2-yl)methyl-1H-pyrrolo [2,3-b]pyridine

1,2,3,4-Tetrahydroisoquinoline (1.37g, 10.0mmol) was dissolved in acetic acid (4ml) and water (2ml). 37% Aqueous formaldehyde (0.9ml, 12mmol) was added and the reaction mixture stirred for five minutes, 1H-pyrrolo[2,3-b]pyridine (1.18g, 9.99mmol) added and the resulting solution stirred at room temperature for 20h. The reaction mixture was poured into 2M aqueous sodium hydroxide solution (50ml) and extracted with ethyl acetate (3 x 50ml). The combined extracts were washed with water (50ml) and brine (50ml). The organic phase was dried (MgSO₄) and concentrated *in vacuo* to give a lemon solid. Recrystallisation from toluene gave the *title compound* (0.935g, 36%), as an off-white solid, m.p. 190-192°C; (Found: C, 77.51; H, 6.50; N, 15.57. C₁₇H₁₇N₃ requires C, 77.54; H, 6.51; N, 15.96%); δ_{H} (DMSO-d₆) 2.68 (2H, t, J 7.8Hz, CH₂), 2.77 (2H, d, J 7.5Hz, CH₂), 3.55 (2H, s, CH₂), 3.79 (2H, s, CH₂), 6.97-7.07 (5H, m, ArH), 7.41 (1H, d, J 1.8Hz, ArH), 8.03 (1H, dd, J 7.8, 1.5Hz, 4-H), 8.20 (1H, dd, J 4.6, 1.5Hz, 6-H), and 11.48 (1H, br s, NH); m/z (CI⁺, NH₃) 264 (M+1)⁺.

Prepared in an analogous manner were:

### EXAMPLE 2

### 3-(4-[2-Phenylethyl]piperidin-1-yl)methyl-1H-pyrrolo[2,3-b] pyridine.

M.p. 162-163°C (PhMe); (Found: C, 79.29; H, 7.97; N, 12.93. C₂₁H₂₅N₃ requires C, 78.96; H, 7.89; N, 13.15%); δ_{H} (DMSO-d₆) 1.15 (3H, m, 3 x piperidinyl H), 1.48 (2H, m, 2 x piperidinyl H), 1.64 (2H, m, CH₂CH₂Ph), 1.85 (2H, t, J 10.2Hz, 2 x piperidinyl H), 2.56 (2H, t, J 8Hz, CH₂CH₂Ph), 2.83 (2H, d, J 11.2Hz, 2 x piperidinyl H), 3.57 (2H, s, CH₂N), 7.02 (1H, dd, J 7.8, 4.6Hz, 5-H), 7.12-7.17 (3H, m, ArH), 7.23-7.30 (3H, m, ArH), 8.00 (1H, dd, J 7.8, 1.5Hz, 4-H), 8.80 (1H, dd, J 4.6, 1.5Hz, 6-H), and 11.39 (1H, br s, NH); m/z (CI⁺, NH₃) 320 (M+1)⁺.

### EXAMPLE 3

### 3-(4-Phenyl-1,2,3,6-tetrahydropyridin-1-yl)methyl-1H-pyrrolo[2,3-b]pyridine

M.p. 177-179°C (MeOH); (Found: C, 78.60; H, 6.80; N, 14.54. C₁₉H₁₉N₃ requires C, 78.86; H, 6.62; N, 14.52%); δ_{H} (DMSO-d₆) 2.45 (2H, br s, CH₂), 2.66 (2H, t, J 5.6Hz, CH₂), 3.09 (2H, d, J 3.2Hz, CH₂), 3.74 (2H, s, CH₂N), 6.15 (1H, m, CH), 7.03 (1H, dd, J 7.8, 4.6Hz, 5-H), 7.19-7.26 (1H, m, ArH), 7.30-7.32 (2H, m, ArH), 7.38-7.41 (3H, m, ArH), 8.03 (1H, dd, J 7.8, 1.5Hz, 4-H), 8.19 (1H, dd, J 4.6, 1.5Hz, 6-H), and 11.46 (1H, br s, NH); m/z (CI⁺, NH₃) 290 (M+1)⁺.

### EXAMPLE 4

### (E)-3-(4-[2-Phenylethenyl]-1,2,3,6-tetrahydropyridin-1-yl)methyl-1H-pyrrolo[2,3-b]pyridine

A solution of (*E*)-4-(2-phenylethenyl)-1,2,3,6-tetrahydropyridine (204mg, 1.1mmol) and 3-dimethylaminomethyl-1H-pyrrolo[2,3-b] pyridine (192mg, 1.1mmol) [prepared by the method of M.M. Robison and B.L. Robison, J. Am. Chem. Soc., 1955, 77, 457] in toluene (15ml) was stirred under reflux for 24h. The hot solution was filtered and the filtrate allowed to cool, giving the *title compound* (196mg, 55%) as a pale brown solid, m.p. 210-212°C; (Found: C, 80.43; H, 6.45; N, 13.18. C₂₁H₂₁N₃O. 0.1PhMe requires: C, 80.31; H, 6.74; N, 12.95%); δ_{H} (CDCl₃) 2.42 (2H, br s, 3'-CH₂), 2.72-2.75 (2H, m, 2'-CH₂), 3.20 (2H, br s, 6'-CH₂), 3.83 (2H, s, CH₂N), 5.82 (1H, br s, CH=CR), 6.44 (1H, d, J 16.1Hz, CH=CHPh), 6.78 (1H, d, J 16.3Hz, CH=CHPh), 7.07-7.10 (1H, m, 5-H), 7.17-7.21 (1H, m, ArH), 7.26-7.31 (3H, m, ArH), 7.38-7.40 (2H, m, ArH), 8.09 (1H, dd, J 7.9, 1.5Hz, 4-H), 8.31 (1H, dd, J 4.8, 1.5Hz, 4-H), and 9.31 (1H, br s, NH); m/z (CI⁺, NH₃) 316 (M+1)⁺.

### EXAMPLE 5

### 3-(4-Phenylethyl-1,2,3,6-tetrahydropyridin-1-yl)methyl-1H-pyrrolo[2,3-b]pyridine

M.p. 121-122°C (MeOH); (Found: C, 79.06; H, 7.28; N, 13.27. C₂₁H₂₃N₃ requires C, 79.46; H, 7.30; N, 13.24%); δ_{H} (DMSO-d₆) 2.02 (2H, br s, tetrahydropyridinyl CH₂), 2.19 (2H, t, J 7.8Hz, CH₂CH₂Ph) 2.49-2.52 (2H, m, CH₂), 2.65 (2H, t, J 8.0Hz, CH₂), 2.85 (2H, s, CH₂), 3.65 (2H, s, NCH₂Ar), 5.34 (1H, s, CH=CR), 7.02 (1H, dd, J 7.8, 4.6Hz, 5-H), 7.13-7.19 (3H, m, ArH), 7.23-7.27 (2H, m, ArH), 7.33 (1H, d, J 2.2Hz, 2-H), 7.99 (1H, dd, J 7.8, 1.3Hz, 4-H), 8.18 (1H, dd, J 4.6, 1.5Hz, 6-H), and 11.42 (1H, br s, NH); m/z (CI⁺, NH₃) 318 (M+1)⁺.

### EXAMPLE 6

### 3-(4-Naphthalen-2-yl-1,2,3,6-tetrahydropyridin-1-yl)methyl-1H-pyrrolo[2,3-b]pyridine

### Step 1: 1-(4-Hydroxy-4-naphthalen-2-yl-piperidin-1-yl)ethanone

2-Bromonaphthalene (24.75g, 0.12mol) in tetrahydrofuran (180ml) was slowly added to magnesium (2.9g, 0.12mol) in tetrahydrofuran (20ml) and the mixture stirred at room temperature for one hour. The resultant solution was slowly added to 1-acetyl-4-piperidone (16.9g, 0.12mol) in tetrahydrofuran (200ml) at -78°C, and then allowed to stir to room temperature. The white suspension formed was hydrolysed with hydrochloric acid (120ml, 1M) and the organic layer removed and concentrated *in vacuo.* The residue was taken up in dichloromethane, dried (MgSO₄), and concentrated to leave a clear oil. Trituration of this oil with diethyl ether gave 1-(4-hydroxy-4-naphthalen-2-yl-piperidin-1-yl)ethanone (13.92g, 43%) as a white solid; δ_{H} (CDCl₃) 1.90 (4H, m, 2 x piperidinyl CH₂), 2.10 (2H, m, piperidinyl CH₂), 2.15 (3H, s, COCH₃), 3.20 (1H, m, piperidinyl H), 3.70 (1H, m, piperidinyl H), 4.60 (1H, br m, OH), 7.50 (2H, m, ArH), 7.60 (1H, dd, J 9.6, 1.6Hz, ArH), and 7.90 (4H, m, ArH).

### Step 2: 4-Naphthalen-2-yl-1,2,3,6-tetrahydropyridine

1-(4-Hydroxy-4-naphthalen-2-yl-piperidin-1-yl)ethanone (3.40g, 13mmol) was dissolved in hydrochloric acid (50ml, 6M) and the solution refluxed for 12 hours. The resultant mixture was cooled in ice, basified with sodium hydroxide solution (32ml, 10M) and extracted with dichloromethane (2 x 100ml). The extracts were combined, dried (MgSO₄) and concentrated to give 4-naphthalen-2-yl-1,2,3,6-tetrahydropyridine (2.12g, 78%) as a tan solid; δ_{H} (CDCl₃) 2.60 (2H, m, tetrahydropyridinyl CH₂), 3.20 (2H, t, J 5.6Hz, tetrahydropyridinyl CH₂), 3.60 (2H, m, tetrahydropyridinyl CH₂), 6.30 (1H, m, CH=CR), 7.45 (2H, m, ArH), 7.60 (1H, dd, J 8.4, 1.4Hz, ArH), and 7.80 (4H, m, ArH).

### Step 3: 3-(4-Naphthalen-2-yl-1,2,3,6-tetrahydropyridin-1-yl)methyl-1H-pyrrolo[2,3-b]pyridine

4-Naphthalen-2-yl-1,2,3,6-tetrahydropyridine (597mg, 2.9mmol) was reacted with 3-dimethylaminomethyl-1H-pyrrolo[2,3-b]pyridine (500mg, 2.9mmol) as exemplified in Example 4 to give the *title compound* (619mg, 63%) as tan crystals, m.p. 223-225°C (EtOH); (Found: C, 81.05; H, 6.24; N, 12.19. C₂₃H₂₁N₃ requires C, 81.38; H, 6.24; N, 12.38%); δ_{H} (DMSO-d₆) 2.60 (2H, m, tetrahydropyridinyl CH₂), 2.72 (2H, t, J 5.7Hz, tetrahydropyridinyl CH₂), 3.16 (2H, d, J 2.7Hz, tetrahydropyridinyl CH₂), 3.78 (2H, s, ArCH₂N), 6.34 (1H, m, CH=CR), 7.04 (1H, dd, J 7.5, 1.5Hz, 5-H), 7.40 (1H, d, J 2.3Hz, ArH), 7.44 (2H, m, ArH), 7.66 (1H, dd, J 1.6, 7.8Hz, ArH), 7.86 (4H, m, ArH), 8.06 (1H, d, J 7.5Hz, 4-H), 8.20 (1H, d, J 1.5Hz, 6-H), and 11.48 (1H, br s, NH); m/z (CI⁺, NH₃) 340 (M+1)⁺.

### EXAMPLE 7

### 3-(4-[4-Methoxyphenyl]-1,2,3,6-tetrahydropyridin-1-yl)methyl-1H-pyrrolo[2,3-b]pyridine

### Step 1: 1-(4-Hydroxy-4-[4-methoxyphenyl]-piperidin-1-yl)ethanone

4-Bromoanisole (22.4g, 0.12mol) in tetrahydrofuran (180ml) was slowly added to magnesium (2.9g, 0.12mol) in tetrahydrofuran (20ml) and the mixture stirred for one hour at room temperature. The solution formed was slowly added to a solution of 1-acetyl-4-piperidone (16.9g, 0.12mol) in tetrahydrofuran (200ml) at -78°C, and the suspension produced was stirred to room temperature for 2 hours. Hydrochloric acid (120ml, 1M) was added and the organic phase was separated and concentrated *in vacuo.* The residue was taken up in dichloromethane (300ml), washed with water (300ml) and dried (MgSO₄). Concentration *in vacuo* and trituration with diethyl ether gave 1-[4-hydroxy-4-(4-methoxyphenyl)-piperidin-1-yl]ethanone (14.75g, 50% yield) as a white solid; δ_{H} (CDCl₃) 1.7-2.1 (4H, m, 2 x piperidinyl CH₂), 2.1 (3H, s, COCH₃), 3.1 (1H, m, piperidinyl H), 3.6-3.8 (3H, m, piperidinyl H), 3.8 (3H, s, ArOCH₃), 4.6 (1H, m, OH), 6.9 (2H, d, J 10Hz, ArH), and 7.4 (2H, d, J 10Hz, ArH).

### Step 2: 4-(4-Methoxyphenyl)-1,2,3,6-tetrahydropyridine

1-(4-Hydroxy-4-[4-methoxyphenyl]piperidin-1-yl)ethanone (14.75g, 59mmol) was dissolved in hydrochloric acid (250mg, 6M) and refluxed for 12 hours. The reaction mixture was cooled in ice and basified with sodium hydroxide solution (155ml, 10M), then extracted with dichloromethane (2 x 500ml). The extractions were combined, dried (MgSO₄), and concentrated *in vacuo.* The crude product was purified by flash chromatography eluting with 10% methanol in dichloromethane, containing 1% aqueous ammonia. Concentration of the appropriate fractions gave 4-(4-methoxyphenyl)-1,2,3,6-tetrahydropyridine (2.65g, 24%) as a tan solid; δ_{H} (DMSO-d₆) 2.3 (2H, m, tetrahydropyridinyl CH₂), 2.9 (2H, t, J 5.6Hz, tetrahydropyridinyl CH₂), 3.4 (2H, d, J 3.4Hz, tetrahydropyridinyl CH₂), 3.7 (3H, s, ArOCH₃), 6.1 (1H, m, CH=CR), 6.9 (2H, d, J 9.6Hz, ArH), 7.3 (2H, d, J 9.6Hz, ArH), and 9.6 (1H, br s, NH).

### Step 3: 3-(4-[4-Methoxyphenyl]-1,2,3,6-tetrahydropyridin-1-yl)methyl-1H-pyrrolo[2,3-b]pyridine

4-(4-Methoxyphenyl)-1,2,3,6-tetrahydropyridine (540mg, 2.8mmol) was reacted with 3-dimethylaminomethyl-1H-pyrrolo[2,3-b]pyridine (500mg, 2.8mmol) as in Example 4 to give the *title compound* (367mg, 41%) as tan crystals, m.p. 202-204°C (iPrOH); (Found: C, 73.51; H, 6.74; N, 12.15. C₂₀H₂₁N₃O.0.33 H₂O.0.2 C₃H₈O requires C, 73.34; H, 6.95; N, 12.45%); δ_{H} (DMSO-d₆) 2.44 (2H, m, tetrahydropyridinyl CH₂), 2.70 (2H, m, tetrahydropyridinyl CH₂), 3.10 (2H, m, tetrahydropyridinyl CH₂), 3.74 (3H, s, ArOCH₃), 3.76 (2H, s, ArCH₂N), 6.04 (1H, m, CH=CR), 6.90 (2H, d, J 8.6Hz, ArH), 7.04 (1H, dd, J 7.7, 4.6Hz, 5-H), 7.36 (2H, d, J 8.5Hz, ArH), 7.40 (1H, s, 2-H), 8.06 (1H, d, J 7.7Hz, 4-H), 8.20 (1H, d, J 4.6Hz, 6-H), and 11.48 (1H, br s, NH); m/z (CI⁺, NH₃) 320 (M+1)⁺.

### EXAMPLE 8

### 3-(4-[1H-Indol-5-yl]-1,2,3,6-tetrahydropyridin-1-yl)methyl-1H-pyrrolo[2,3-b]pyridine

### Step 1: 5-Bromo-1-triisopropylsilyl-1H-indole

5-Bromoindole (14.04g, 72mmol) in tetrahydrofuran (50ml) was added dropwise to a suspension of sodium hydride (60% dispersion in oil; 3.04g, 76mmol) in tetrahydrofuran (100ml) and the resultant solution stirred for one hour at room temperature. Triisopropylsilyltrifluoromethane sulphonate (23.3g, 76mmol) was added slowly and the solution stirred for a further one hour. The reaction mixture was washed with sodium hydrogen carbonate solution, dried (MgSO₄), and concentrated. The crude mixture was purified by flash chromatography, with hexane as eluant, to give 5-bromo-1-triisopropylsilyl-1H-indole (20.0g, 79%) as a clear oil; δ_{H} (CDCl₃) 1.10 (18H, d, J 8.4Hz, 6 x CH₃), 2.70 (3H, m, 3 x CH), 6.55 (1H, d, J 3.9Hz, ArH), 7.20 (2H, m, ArH), 7.35 (1H, d, J 8.5Hz, ArH), and 7.75 (1H, d, J 1.4Hz, ArH).

### Step 2: 1-[4-Hydroxy-4-(1-triisopropylsilyl-1H-indol-5-yl)piperidin-1-yl]ethanone

5-Bromo-1-triisopropylsilyl-1H-indole (13.7g, 38.9mmol) in tetrahydrofuran (250ml) at -78°C was treated with t-butyllithium (45.8ml, 77.8mmol, 1.7M solution in pentanes) and stirred at this temperature for 15 minutes. The yellow solution formed was added to 1-acetyl-4-piperidone (5.49g, 38.9mmol) in tetrahydrofuran (250ml) at -78°C, and the mixture allowed to warm to room temperature. Hydrochloric acid (40ml, 1M) was added and the mixture extracted with ethyl acetate (2 x 500ml). The extracts were combined, dried (MgSO₄) and concentrated. The crude product was purified by flash chromatography using ethyl acetate as eluant, to give 1-[4-hydroxy-4-(1-triisopropylsilyl-1H-indol-5-yl)piperidin-1-yl]ethanone (3.39g, 21%) as a white solid; δ_{H} (DMSO-d₆) 1.10 (18H, d, J 7.9Hz, 6 x CH₃), 1.70 (3H, m, 3 x CH), 2.00 (2H, m, piperidinyl H), 2.05 (3H, s, COCH₃), 2.45 (1H, m, piperidinyl H), 3.00 (1H, m, piperidinyl H), 3.30 (1H, m, piperidinyl H), 3.50 (1H, m, piperidinyl H), 3.70 (1H, m, piperidinyl H), 4.30 (1H, m, piperidinyl H), 5.00 (1H, s, OH), 6.60 (1H, d, J 2.8Hz, ArH), 7.30 (1H, d, J 7.8Hz, ArH), 7.35 (1H, d, J 2.2Hz, ArH), 7.50 (1H, d, J 7.9Hz, ArH), and 7.70 (1H, s, ArH).

### Step 3: 1-(4-[1-Triisopropylsilyl-1H-indol-5-yl]-1,2,3,6-tetrahydropyridin-1-yl)ethanone

1-(4-Hydroxy-4-[1-triisopropylsilyl-1H-indol-5-yl]-piperidin-1-yl)ethanone (3.11g, 7.4mmol) in dichloromethane (100ml) was treated with pyridinium *p*-toluenesulfonate (3.8g, 15.1mmol) and stirred for 72 hours. The solution was poured into water and extracted with dichloromethane (100ml). The extractions were dried (MgSO₄), concentrated and the crude product purified by flash chromatography using ethyl acetate as eluant, to give 1-(4-[1-triisopropylsilyl-1H-indol-5-yl]-1,2,3,6-tetrahydropyridin-1-yl)-ethanone (1.95g, 66%) as a white solid; δ_{H} (CDCl₃) 0.80 (18H, d, J 8.5Hz, 6 x CH₃), 1.30 (3H, m, 3 x CH), 1.75 (3H, d, J 6.7Hz, COCH₃), 2.30 (2H, m, tetrahydropyridinyl H), 3.25 (1H, t, J 5.6Hz, tetrahydropyridinyl H), 3.40 (1H, t, J 5.6Hz, tetrahydropyridinyl H), 3.75 (1H, m, tetrahydropyridyl H), 3.85 (1H, m, tetrahydropyridyl H), 5.60 (1H, m, CH=CR), 6.20 (1H, d, J 2.2Hz, ArH), 6.80 (2H, m, ArH), 7.00 (1H, d, J 7.3Hz, ArH), and 7.20 (1H, m, ArH).

### Step 4: 5-(1,2,3,6-Tetrahydropyridin-4-yl)-1H-indole

1-(4-[1-Triisopropylsilyl-1H-indol-5-yl]-1,2,3,6-tetrahydro-2H-pyridin-1-yl)ethanone (1.95g, 4.9mmol) was dissolved in methanol (70ml), treated with potassium hydroxide solution (35ml, 40%), and the mixture heated at 60°C for 12 hours. The reaction mixture was cooled, extracted with ethyl acetate (2 x 100ml), and the extracts were combined, dried (MgSO₄), and concentrated. The crude product was purified by flash chromatography on silica, using 10% methanol in dichloromethane containing 1% ammonia as eluant, to give 5-(1,2,3,6-tetrahydropyridin-4-yl)-1H-indole (619mg, 64%) as a yellow solid; δ_{H} (CDCl₃) 2.10 (2H, m, tetrahydropyridinyl CH₂), 2.60 (2H, m, tetrahydropyridinyl CH₂), 3.00 (2H, m, tetrahydropyridinyl CH₂), 5.50 (1H, m, CH=CR), 5.90 (1H, s, ArH), 6.60-7.10 (4H, m, ArH).

### Step 5: 3-(4-[1H-Indol-5-yl]-1,2,3,6-tetrahydropyridin-1-yl)methyl-1H-pyrrolo[2,3-b]pyridine

5-(1,2,3,6-Tetrahydropyridin-4-yl)-1H-indole (200mg, 1.01mmol) and 3-dimethylaminomethyl-1H-pyrrolo[2,3-b]pyridine (177mg, 1.01mmol) were coupled as described in Example 4 to give a solid which was purified by flash chromatography, eluting with 10% methanol in dichloromethane containing 1% ammonia. Recrystallisation from methanol gave the *title compound* (115mg, 35%) as a tan solid, m.p. 187-189°C; (Found: C, 73.78; H, 6.34; N, 16.13. C₂₁H₂₀N₄.0.75 H₂O requires C, 73.77; H, 6.34; N, 16.39%); δ_{H} (DMSO-d₆) 2.68 (2H, m, tetrahydropyridinyl H), 3.10 (2H, s, tetrahydropyridinyl H), 3.18 (1H, d, J 4.4Hz, tetrahydropyridinyl H), (other tetrahydropyridinyl H obscured by solvent at 3.30), 3.74 (2H, s, ArCH₂N), 6.02 (1H, m, CH=CR), 6.40 (1H, s, ArH), 7.04 (1H, dd, J 7.5, 3.2Hz, 5-H), 7.20 (1H, d, J 8.2Hz, ArH), 7.32 (2H, m, ArH), 7.40 (1H, s, ArH), 7.52 (1H, s, ArH), 8.04 (1H, d, J 7.5Hz, 4-H), 8.18 (1H, d, J 3.2Hz, 6-H), 11.00 (1H, br s, NH), and 11.48 (1H, br s, NH); m/z (CI⁺, NH₃) 329 (M+1)⁺.

### EXAMPLE 9

### 3-(4-Benzofuran-5-yl-1,2,3,6-tetrahydropyridin-1-yl)methyl-1H-pyrrolo[2,3-b]pyridine

### Step 1: 5-Bromobenzofuran

A solution of 4-bromophenol (17.3 g, 100 mmol) in dimethylformamide (40 ml) was treated with bromoacetaldehyde dimethyl acetal (16.9 g, 100 mmol) and potassium carbonate (13.8 g, 100 mmol) and heated at 150 °C for 16 h. The mixture was cooled, poured into water (500 ml) and extracted with diethyl ether (3 x 100 ml). The combined organic extracts were dried (MgSO₄), concentrated, and filtered through a plug of silica gel eluting with 10% diethyl ether in hexane. The resulting oil (15 g) was added to a solution of polyphosphoric acid (-12 g) in toluene (100 mL) and heated at 80 °C for 16 h. The mixture was cooled and the toluene layer was decanted off and washed with saturated aqueous sodium hydrogen carbonate, dried (MgSO₄), concentrated and the residue distilled to give 5-bromobenzofuran (6 g) as a colourless oil, bp. 98°C / 1 mmHg; δ_{H} (DMSO-d₆) 6.96 (1H, m, ArH), 7.45 (1H, m, ArH), 7.59 (1H, m, ArH), 7.91 (1H, m, ArH), and 8.05 (1H, m, ArH).

### Step 2: 1-(4-Benzofuran-5-yl-4-hydroxypiperidin-1-yl)ethanone

The Grignard of 5-bromobenzofuran (1.54 g, 7.8 mmol) was prepared from magnesium (209 mg, 8.6 mmol) in tetrahydrofuran (10 ml), initiating the reaction with 1,2-dibromoethane. The Grignard was added to a solution of *N*-acetyl-4-piperidone (1.21 g, 8.6 mmol) in tetrahydrofuran at -78°C resulting in a thick precipitate. The mixture was warmed to room temperature, digested with 2 M hydrochloric acid (10ml) and extracted with dichloromethane (3 x 50ml). The combined organic extracts were dried (MgSO₄), concentrated, and purified by flash chromatography, eluting with ethyl acetate, to give the *title product* as a colourless oil (750 mg); δ_{H} (DMSO-d₆) 1.66 (1H, m, piperidinyl H), 1.80 (1H, m, piperidinyl H), 2.03 (1H, m, piperidinyl H), 2.04 (3H, s, CH₃CO), 2.95 (1H, m, piperidinyl H), 3.30 (1H, m, piperidinyl H), 3.40 (1H, m, piperidinyl H), 3.70 (1H, m, piperidinyl H), 4.32 (1H, m, piperidinyl H), 5.14 (1H, s, OH), 6.93 (1H, m, ArH), 7.44 (1H, m, ArH), 7.51 (1H, m, ArH), 7.75 (1H, m, ArH), and 7.95 (1H, m, ArH).

### Step 3: 4-Benzofuran-5-yl-1,2,3,6-tetrahydropyridine

A solution of 1-(4-benzofuran-5-yl-4-hydroxypiperidin-1-yl)ethanone (650 mg, 2.51 mmol) in tetrahydrofuran (10ml) was treated with 6 M HCl (10 mL) and heated at 60 °C for 16 h. The mixture was poured into saturated aqueous sodium hydrogen carbonate (100ml) and extracted with CH₂Cl₂ (3 x 100ml). The combined organic extracts were dried (MgSO₄) and concentrated to give the *title product* (220 mg) as a semi-solid; δ_{H} (DMSO-d₆) 1.4 - 3.7 (6H, 3 x CH₂), 6.16 (1H, m, CH=CR), 6.95 (1H, m, ArH), 7.45 (1H, m, ArH), 7.59 (1H, m, ArH), 7.72 (1H, m, ArH), and 7.99 (1H, m, ArH).

### Step 4: 3-(Benzofuran-5-yl-1,2,3,6-tetrahydropyridin-1-yl)methyl-1H-pyrrolo[2,3-b]pyridine

A suspension of 7-azagramine (180 mg, 1.0 mmol) and 4-benzofuran-5-yl-1,2,3,6-tetrahydropyridine (200 mg, 1.0 mmol) in toluene (5ml) was heated at 110 °C for 16 h. The resulting hot solution was filtered and allowed to cool. The *title compound* (180 mg) crystallised and was collected by filtration as a white, crystalline solid, m.p. 168-70°C; (Found: C, 75.02; H, 5.81; N, 12.28. C₂₁H₁₉N₃O.0.33.H₂O requires C, 75.20; H, 5.91; N, 12.53); δ_{H} (DMSO-d₆) 2.50 (2H, br s, tetrahydropyridinyl CH₂, obscured by solvent), 2.69 (2H, br s, CH₂), 3.11 (2H, br s, tetrahydropyridinyl CH₂), 3.76 (2H, s, ArCH₂N), 6.12 (1H, br s, CH=CR), 6.91 (1H, s, ArH), 7.03 (1H, m, ArH), 7.40 (2H, m, ArH), 7.51 (1H, m, ArH), 7.64 (1H, s, ArH), 7.95 (1H, m, ArH), 8.04 (1H, m, ArH), 8.20 (1H, m, ArH), and 11.47 (1H, br s, NH); m/z (CI⁺, NH₃) 330 (M+H)⁺.

### EXAMPLE 10

### 3-(4-Benzofuran-6-yl-1,2,3,6-tetrahydropyridin-1-yl)methyl-1H-pyrrolo[2,3-b]pyridine

A suspension of 7-azagramine (551 mg, 3.15 mmol) and a 1 : 1 mixture of 4-benzofuran-6-yl-1,2,3,6-tetrahydropyridine and 4-benzofuran-4-yl-1,2,3,6-tetrahydropyridine (645 mg, 3.24 mmol, produced from 3-bromophenol following the analogous procedure described in Example 9) in toluene (10ml) was heated at 110 °C for 16 h. The resulting hot solution was filtered and cooled. An isomeric mixture of products crystallised from solution and was collected by filtration as a white crystalline solid. The *title compound* was isolated pure as its trifluoroacetate salt (60 mg) by preparative reversed phase HPLC (C₁₈ column, eluting with 30% MeCN in 1% aqueous trifluoroacetic acid, required product is the faster eluting of the two products), m.p. 161 - 163 °C (Et₂O); (Found: C, 59.73; H, 4.35; N, 9.13. C₂₁H₁₉N₃O.CF₃CO₂H.H₂O requires C, 59.87; H, 4.81; N, 9.11); δ_{H} (DMSO-d₆) 2.86 (2H, br s, tetrahydropyridinyl CH₂), 3.38 (1H, m, tetrahydropyridinyl H), 3.74 (1H, m, tetrahydropyridinyl H), 3.89 (2 H, br s, tetrahydropyridinyl H), 4.60 (2H, br s, ArCH₂N), 6.25 (1H, br s, CH=CR), 6.95 (1H, s, ArH), 7.21 (1H, m, ArH), 7.40 (1H, m, ArH), 7.64 (1H, m, ArH), 7.70 (1H, s, ArH), 7.76 (1H, br s, ArH), 8.00 (1H, br s, ArH), 8.25 (1H, m, ArH), 8.31 (1H, m, ArH), and 9.88 (1H, br s, NH⁺), and 12.07 (1H, bs, NH); m/z (CI⁺, NH₃) 330 (M+H)⁺.

### EXAMPLE 11

### 3-(4-[Benzothiophen-2-yl]-1,2,3,6-tetrahydropyridin-1-yl)methyl-1H-pyrrolo[2,3-b]pyridine

### Step 1: 1-Benzyl-4-(benzothiophen-2-yl)-1,2,3,6-tetrahydropyridine

To a solution of benzothiophene (3g, 22.4mmol) in anhydrous tetrahydrofuran (50ml) at -10°C under nitrogen was added n-butyllithium (9.83ml of a 2.5M solution in toluene), the mixture was allowed to warm to room temperature and stirred for 1 hr. The reaction mixture was cooled to -40°C and 1-benzyl-4-piperidone (4.23g, 22.4mmol) added, allowed to warm to room temperature and stirred for 14 hr. The reaction mixture was concentrated *in vacuo* and trifluoroacetic acid (10ml) added. This mixture was stirred at room temperature for 14 hr and then concentrated *in vacuo.* The product was extracted into dichloromethane (3 x 100ml) from aqueous potassium carbonate. The organic layer was washed with water (1 x 50ml), brine (1 x 50ml) and dried (MgSO₄). After concentration of the extracts the crude product was purified using silica gel column chromatography to yield the *title compound* (4.2g, 68%) as a colourless oil; δ_{H} (CDCl₃) 2.64 (2H, m, tetrahydropyridinyl CH₂), 2.74 (2H, m, tetrahydropyridinyl CH₂), 3.18 (2H, m, tetrahydropyridinyl CH₂), 3.65 (2H, s, PhCH₂N), 6.18 (1H, m, CH=CR), 7.11 (1H, s, 3'-H), and 7.24-7.73 (9H, m, Ar-H).

### Step 2: 4-(Benzothiophen-2-yl)-1,2,3,6-tetrahydropyridine Hydrochloride

To a solution of 1-benzyl-4-(benzothiophen-2-yl)-1,2,3,6-tetrahydropyridine (4g, 13.1mmol) in anhydrous dichloromethane (50ml) at 0°C under nitrogen was added 2-chloroethylchloroformate (1.84ml, 17.0mmol) and the mixture stirred for 1 hr. The reaction mixture was concentrated *in vacuo* and methanol (20ml) added and the solution heated to reflux for 1 hr. After cooling the *title compound* was collected by filtration (2.2g, 67%), m.p. 269°C (dec.).

### Step 3: 3-(4-(Benzothiophen-2-yl)-1,2,3,6-tetrahydropyridin-1-yl)methyl-1H-pyrrolo[2,3-b]pyridine

Reaction of 3-dimethylaminomethyl-1H-pyrrolo[2,3-b]pyridine and 4-(benzothiophen-2-yl)-1,2,3,6-tetrahydropyridine in an analogus manner to Example 4 gave the *title compound,* m.p. 241-246°C (dec.); (Found: C, 72.85; H, 5.54; N, 12.06; C₂₁H₁₉N₃S requires C, 73.01; H, 5.54; N, 12.16%); δ_{H} (DMSO-d₆) 2.51 (2H, br s, tetrahydropyridinyl CH₂), 2.67 (2H, m, tetrahydropyridinyl CH₂), 3.14 (2H, m, tetrahydropyridinyl CH₂), 3.76 (2H, s, CH₂N), 6.19 (1H, br s, CH=CR), 7.03 (1H, m, ArH), 7.31 (2H, m, 3'-H and ArH), 7.74 (1H, d, J 8Hz, ArH), 7.86 (1H, d, J 8Hz, ArH), 8.05 (1H, d, J 8Hz, ArH), 8.20 (1H, m, ArH), and 11.45 (1H, br s, NH); m/z (CI⁺, NH₃) 346 (M+1)⁺.

### EXAMPLE 12

### 3-(4-[Benzofuran-2-yl]-1,2,3,4-tetrahydropyridin-1-yl)methylpyrrolo[2,3-b]pyridine

### Step 1: 4-(Benzofuran-2-yl)-1-benzyl-4-hydroxypiperidine

To a solution of benzofuran (4g, 33.9mmol) in tetrahydrofuran (90ml) at -10°C was slowly added a solution of n-butyllithium (2.5M in hexanes, 13.6ml, 34mmol), keeping the temperature at -10°C. The reaction was stirred and allowed to warm to room temperature over 1 hr, after which a solution of 1-benzyl-4-piperidone (6.3ml, 34mmol) in tetrahydrofuran (30ml) was added dropwise. Stirring was continued for 2 hr at room temperature. The solvent was evaporated and the residue partitioned between saturated aqueous sodium carbonate (75ml) and ethyl acetate (3 x 100ml). The combined organics were dried (MgSO₄) and evaporated. The residue was purified by column chromatography, eluting with ethyl acetate, to give 4-(benzofuran-2-yl)-1-benzyl-4-hydroxypyridine (7.5g, 75%); δ_{H} (CDCl₃) 1.96-2.00 (2H, m, piperidinyl CH₂), 2.21-2.29 (2H, m, piperidinyl CH₂), 2.44 (1H, t, J 6.2Hz, piperidinyl CH), 2.56 (2H, dt, J 11.1, 2.7Hz, piperidinyl CH₂), 2.64-2.68 (3H, m, piperidinyl CH₂, OH), 2.73 (1H, t, J 6.2Hz, piperidinyl CH), 3.55 (2H, s, PhCH₂N), 6.58 (1H, s, 3'-H), 7.19-7.34 (7H, m, ArH), 7.44 (1H, d, J 7.3Hz, ArH), and 7.52 (1H, d, J 6.6Hz, ArH).

### Step 2: 2-(1-Benzyl-1,2,3,6-tetrahydropyridin-4-yl)benzofuran

A mixture of the preceding alcohol (2g, 6.5mmol) and 4-toluenesulfonic acid monohydrate (1.2g, 6.6mmol) in toluene (100ml) was refluxed under Dean and Stark conditions for 2 hrs. The solvent was evaporated, and the residue partitioned between dichloromethane (3 x 100ml) and saturated aqueous sodium carbonate (100ml). The combined organics were dried (MgSO₄) and evaporated to yield the *title compound* (1.2g, 64%). An analytical sample was recrystallised from ether to give 2-(1-benzyl-1,2,3,4-tetrahydropyridin-4-yl)benzofuran, m.p. 139-140°C; (Found: C, 82.40; H, 6.43; N, 4.67. C₂₀H₁₉NO.0.1H₂O requires C, 82.50; H, 6.65; N, 4.81%); δ_{H} (CDCl₃) 2.55 (2H, br s, tetrahydropyridinyl CH₂), 2.74 (2H, t, J 5.7Hz, tetrahydropyridinyl CH₂), 3.24 (2H, br s, tetrahydropyridinyl CH₂), 3.67 (2H, s, NCH₂Ph), 6.48-6.52 (2H, m, tetrahydropyridinyl 5-CH, 3'-H), 7.14-7.42 (8H, m, ArH), and 7.49 (1H, d, J 7.5Hz, ArH); m/z (CI⁺, NH₃), 290 (M+1)⁺.

### Step 3: 4-(Benzofuran-2-yl)-1,2,3,6-tetrahydropyridine

To a solution of the foregoing tetrahydropyridine (1.1g, 3.8mmol) in dichloromethane (50ml) at 0°C was added 1-chloroethylchloroformate (493µl, 4.6mmol), keeping the temperature below 0°C. The reaction was allowed to warm to room temperature over 1 hr, after which the solvent was evaporated. Methanol (50ml) was added to the residue and the mixture heated under reflux for 2 hrs. The solvent was removed by evaporation, and the residue chromatographed on silica, eluting with dichloromethane/methanol/ammonia (90:10:1), to yield the *title compound* as a buff solid (600mg, 79%); δ_{H} (CDCl₃) 2.44-2.48 (2H, m, tetrahydropyridinyl CH₂), 3.14 (2H, t, J 5.8Hz, tetrahydropyridinyl CH₂), 3.60-3.63 (2H, m, tetrahydropyridinyl CH₂), 6.54-6.58 (2H, m, CH=CR, 3'-H), 7.16-7.26 (2H, m, 5'-H, 6'-H), 7.42 (1H, d, J 7.8Hz, 7'-H), and 7.50 (1H, d, J 7.2Hz, 4'-H).

### Step 4: 3-(4-[Benzofuran-2-y]-1,2,3,6-tetrahydropyridin-1-yl)methylpyrrolo[2,3-b]pyridine

Prepared from 4-(benzofuran-2-yl)-1,2,3,6-tetrahydropyridine by the method outlined in Example 4; m.p. 202-204°C (DMF/MeOH); (Found: C, 76.12; H, 5.78; N, 12.46. C₂₁H₁₉N₃O.0.1H₂O requires C, 76.16; H, 5.84; N, 12.69%); δ_{H} (DMSO-d₆) 2.46 (2H, br s, tetrahydropyridinyl CH₂), 2.68 (2H, t, J 5.6Hz, tetrahydropyridinyl CH₂), 3.16 (2H, br s, tetrahydropyridinyl CH₂), 3.77 (2H, s, NCH₂Ar), 6.44 (1H, br s, tetrahydropyridinyl 5-CH), 6.78 (1H, s, 3'-H), 7.01-7.05 (1H, m, ArH), 7.19-7.27 (2H, m, ArH), 7.40 (1H, s, 2-H), 7.50 (1H, d, J 8.3Hz, ArH), 7.57 (1H, d, J 8.3Hz, ArH), 8.04 (1H, dd, 7.8, 1.2Hz, 4-H), 8.20 (1H, dd, J 4.6, 1.5Hz, 6-H), and 11.48 (1H, br s, NH); m/z (CI⁺, NH₃) 330 (MH)⁺.

### EXAMPLE 13

### 3-[4-Phenylethynyl-1,2,3,6-tetrahydropyridin-1-yl]methylpyrrolo[2,3-b]pyridine

M.p. 198-200°C (MeOH); (Found: C, 80.23; H, 6.01; N, 13.03. C₂₁H₁₉N₃ requires C, 80.48; H, 6.11; N, 13.41%); δ_{H} (DMSO-d₆) 2.25 (2H, br s, tetrahydropyridinyl CH₂), 2.58 (2H, t, J 5.6Hz, tetrahydropyridinyl CH₂), 3.16-3.17 (2H, m, tetrahydropyridinyl CH₂), 3.73 (2H, s, NCH₂Ar), 6.14 (1H, br s, tetrahydropyridinyl 5-CH), 7.02-7.05 (1H, m, ArH), 7.34-7.42 (6H, m, ArH), 8.02 (1H, d, J 6.5Hz, ArH), 8.19 (1H, dd, J 4.7, 1.6Hz, 6-H), and 11.44 (1H, br s, NH); m/z (CI⁺, NH₃) 314 (M+1)⁺.

### EXAMPLE 14

### (E)-3-(4-[2-(Thien-3-yl)ethenyl]-1,2,3,6-tetrahydropyridin-1-yl)methylpyrrolo[2,3-b]pyridine

M.p. 215-217°C (MeOH); (Found: C, 71.15; H, 5.92; N, 12.78. C₁₉H₁₉N₃S requires C, 70.99; H, 5.96; N, 13.07%); δ_{H} (DMSO-d₆) 2.26 (2H, br s, tetrahydropyridinyl CH₂), 2.61 (2H, t, J 5.6Hz, tetrahydropyridinyl CH₂), 3.05 (2H, br s, tetrahydropyridinyl CH₂), 3.72 (2H, s, NCH₂Ar), 5.81 (1H, br s, tetrahydropyridinyl 5-CH), 6.47 (1H, d, J 16.2Hz, CCH=CH), 6.72 (1H, d, J 16.2Hz, CH=CHAr), 7.01-7.04 (1H, m, ArH), 7.33-7.37 (2H, m, ArH), 7.42 (1H, br s, ArH), 7.48-7.50 (1H, m, ArH), 8.02 (1H, d, J 6.7Hz, ArH), and 8.19 (1H, dd, J 4.6, 1.4Hz, 6-H); m/z (CI⁺, NH₃) 322 (M+1)⁺, 339 (M+NH₄⁺).

### EXAMPLE 15

### (E)-3-(4-[2-(2-Chlorophenyl)ethenyl]-1,2,3,6-tetrahydropyridin-1-yl)methylpyrrolo[2,3-b]pyridine

M.p. 174-176°C (xylene); (Found: C, 71.38; H, 5.62; N, 11.70. C₂₁H₂₀ClN₃.0.2H₂O requires C, 71.36; H, 5.82; N, 11.89%); δ_{H} (DMSO-d₆) 2.31 (2H, br s, tetrahydropyridinyl CH₂), 2.64 (2H, br s, tetrahydropyridinyl CH₂), 3.08 (2H, br s, tetrahydropyridinyl CH₂), 3.74 (2H, s, NCH₂Ar), 5.98 (1H, br s, tetrahydropyridinyl 5-CH), 6.70 (1H, d, J 16.1Hz, CH=CHAr), 6.94 (1H, d, J 16.1Hz, CH=CHAr), 7.01-7.05 (1H, m, ArH), 7.23-7.32 (2H, m, ArH), 7.37-7.43 (2H, m, ArH), 7.72 (1H, d, J 6.7Hz, ArH), 8.03 (1H, d, J 7.7Hz, ArH), 8.19 (1H, br s, ArH), and 11.46 (1H, br s, NH); m/z (CI⁺, NH₃) 350 (M+1)⁺.

### EXAMPLE 16

### (E)-3-(4-[2-(4-Chlorophenyl)ethenyl]-1,2,3,6-tetrahydropyridin-1-yl)methylpyrrolo[2,3-b]pyridine

M.p. 214-217°C (xylene); (Found: C, 71.34; H, 5.87; N, 11.60. C₂₁H₂₀ClN₃.0.2H₂O requires C, 71.36; H, 5.82; N, 11.89%); δ_{H} (DMSO-d₆) 2.29 (2H, br s, tetrahydropyridinyl CH₂), 2.61 (2H, t, J 5.7Hz, tetrahydropyridinyl CH₂), 3.07 (2H, br s, tetrahydropyridinyl CH₂), 3.73 (2H, s, NCHAr), 5.91 (1H, br s, tetrahydropyridinyl 5-CH), 6.44 (1H, d, J 16.3Hz, CH=CHAr), 6.90 (1H, d, J 16.3Hz, CH=CHAr), 7.01-7.04 (1H, m, ArH), 7.34-7.38 (3H, m, ArH), 7.48 (2H, d, J 8.5Hz, ArH), 8.02 (1H, d, J 7.8Hz, ArH), 8.15-8.19 (1H, m, ArH), and 11.45 (1H, bs, NH); m/z CI⁺, NH₃) 350 (M+1)⁺.

### EXAMPLE 17

### (E)-3-(4-[2-(Thien-2-yl)ethenyl]-1,2,3,6-tetrahydropyridin-1-yl)methylpyrrolo[2,3-b]pyridine

M.p. 202-204°C (MeOH); (Found: C, 70.59; H, 5.94; N, 12.20. C₁₉H₁₉N₃S requires C, 70.99; H, 5.96; N, 13.07%); δ_{H} (DMSO-d₆) 2.26 (2H, br s, tetrahydropyridinyl CH₂), 2.60 (2H, t, J 5.7Hz, tetrahydropyridinyl CH₂), 3.05 (2H, br s, tetrahydropyridinyl CH₂), 3.72 (2H, s, NCH₂Ar), 5.85 (1H, br s, tetrahydropyridinyl 5-H), 6.58 (1H, d, J 16.1Hz, CH=CHAr), 6.64 (1H, d, J 16.1Hz, CH=CHAr), 6.98-7.07 (3H, m, ArH), 7.37 (2H, br s, ArH), 8.02 (1H, d, J 6.4Hz, ArH), 8.19 (1H, d, J 3.1Hz, ArH), and 11.42 (1H, br s, NH).

### EXAMPLE 18

### 3-(4-[2-(Furan-2-yl)ethyl]-1,2,3,6-tetrahydropyridin-1-yl)methyl-1H-pyrrolo[2,3-b]pyridine

### Step 1: 1-(Pyridin-4-yl)-2-(furan-2-yl)ethene

A solution of 4-methylpyridine (15g, 0.16mol) in acetic anhydride (100ml) was treated with 2-furaldehyde (15.5g, 0.16mol) and heated at reflux for 16 hr. The solvent was evaporated to give a black oil which was treated with water (30ml) and stirred for 30 mins at room temperature. Ethyl acetate (150ml) and saturated sodium carbonate (100ml) were then added and the stirring continued for 30 mins. The supernatant was decanted to leave a black oily residue which was retained (A). From the decanted solvents the organic phase was separated, dried (Na₂SO₄) and evaporated to give a black oil (B). The oily residue (A) was dissolved in dichloromethane (150ml), washed with saturated sodium carbonate solution (100ml), dried (Na₂SO₄) and evaporated to give a black oil which was combined with oil (B). The mixture was chromatographed on silica gel with a gradient of ethyl acetate in hexane (50% - 100%) as eluant to afford the *title compound* as a brown solid (9.8g, 36%); δ_{H} (DMSO-d₆) 6.58-6.64 (1H, m, furanyl H), 6.66-6.72 (1H, m, furanyl H), 6.96 (1H, d, J 17.5Hz, ArCH=CHAr'), 7.42 (1H, d, J 17.5Hz, ArCH=CHAr'), 6.50-6.58 (2H, m, pyridinyl CH), 7.78-7.82 (1H, m, furanyl H), and 8.50-8.51 (2H, m, pyridinyl H).

### Step 2: 1-(Pyridin-4-yl)-2-(furan-2-yl)ethane

A solution of 1-(pyridin-4-yl)-2-(furan-2-yl)ethene (8g, 46.8mmol) in methanol (200ml) was treated with ammonium formate (14.7g, 234.0mmol). 10% Palladium on charcoal catalyst (400mg, 5% (w/w)) was added and the mixture was stirred at reflux for five hours. The catalyst was filtered off and the solvent evaporated. The residue was partitioned between dichloromethane and water. The organic layer was separated, dried (MgSO₄) and evaporated to give a beige oil. This material was chromatographed on silica gel with a gradient of ethyl acetate in hexane (50%-100%) as eluant to afford the *title compound* as a colourless oil (2.5g, 31%); δ_{H} (DMSO-d₆) 2.94-3.06 (4H, m, 2 x CH₂), 6.06-6.12 (1H, m, furanyl H), 6.32-6.38 (1H, m, furanyl H), 7.20-7.28 (2H, m, pyridinyl H), 7.50-7.56 (1H, m, furanyl H), and 8.40-8.50 (2H, m, pyridinyl H).

### Step 3: 1-Benzyl-4-(2-[furan-2-yl]ethyl)-1,2,3,6-tetrahydropyridine

A solution of 1-(pyridin-4-yl)-2-(furan-2-yl)ethane (2g, 11.6mmol) in anhydrous dimethylformamide (5ml) was treated with benzyl bromide (1.5ml, 12.7mmol) and the reaction stirred at room temperature for one hour. The reaction was diluted with ethanol (50ml), treated with sodium borohydride (0.55g, 14.5mmol) and heated at reflux for one hour. The solvent was evaporated and the residue partitioned between ethyl acetate and water. The organic was separated, dried (Na₂SO₄) and evaporated to give crude product as a yellow oil. This material was triturated with diethyl ether to afford the *title compound* (2.3g, 68%) as a colourless solid; δ_{H} (DMSO-d₆) 1.92-2.10 (2H, m, tetrahydropyridinyl CH₂), 2.14-2.30 (2H, m, CH₂), 2.36-2.50 (2H, m, CH₂), 2.60-2.82 (2H, m, tetrahydropyridinyl CH₂), 2.84-2.90 (2H, m, tetrahydropyridinyl CH₂), 3.50 (2H, br s, PhCH₂N), 5.32-5.44 (1H, m, tetrahydropyridinyl 5-H), 6.02-6.10 (1H, m, furanyl H), 6.30-6.36 (1H, m, furanyl H), 7.16-7.36 (5H, m, ArH), and 7.44-7.52 (1H, m, furanyl H); m/z (CI⁺, NH₃) 268 (M+1)⁺.

### Step 4: 4-(2-[Furan-2-yl]ethyl)-1,2,3,5-tetrahydropyridine

A cooled (0°C) solution of 1-benzyl-4-(2-[furan-2-yl]ethyl)-1,2,3,6-tetrahydropyridine (1.8g, 6.8mmol) in anhydrous dichloromethane (20ml) was treated with 1-chloroethylchloroformate (0.95ml, 8.8mmol) dropwise. The mixture was stirred for 1 hr at 0°C. The solvent was evaporated and the residue dissolved in methanol (60ml). This solution was heated at reflux for one hour whereupon the solvent was evaporated. The residue was partitioned between dichloromethane and saturated aqueous potassium carbonate solution. The organic phase was separated, dried (Na₂SO₄) and evaporated to give the *title compound* (867mg, 72%) as a colourless oil; δ_{H} (DMSO-d₆) 1.84-1.96 (2H, m, tetrahydropyridinyl CH₂), 2.16-2.30 (2H, m, CH₂), 2.64-2.80 (4H, m, tetrahydropyridinyl CH₂ and CH₂), 3.06-3.10 (2H, m, tetrahydropyridinyl CH₂), 5.40-5.46 (1H, m, tetrahydropyridinyl 5-H), 6.06-6.12 (1H, m, furanyl H), 6.32-6.38 (1H, m, furanyl H), and 7.48-7.56 (1H, m, furanyl H).

### Step 5: 3-(4-[2-(Furan-2-yl)ethyl]-1,2,3,6-tetrahydropyridin-1-yl)methyl-1H-pyrrolo[2,3-b]pyridine

A solution of 4-(2-[furan-2-yl]ethyl)-1,2,3,6-tetrahydropyridine (506mg, 2.86mmol) and 3-dimethylaminomethyl-1H-pyrrolo[2,3-b]pyridine (500mg, 2.86mmol) in toluene (10ml) was stirred at reflux for 10 hours. The solvent was evaporated and the residue chromatographed with 3% methanol in dichloromethane as eluant to afford the *title compound* (200mg, 23%) as a colourless solid, m.p. 105°C; (Found: C, 73.84; H, 6.72; N, 13.31. C₁₉H₂₁N₃O requires C, 74.24; H, 6.89; N, 13.67); δ_{H} (DMSO-d₆) 2.00-2.06 (2H, m, tetrahydropyridinyl CH₂), 2.22 (2H, t, J 7.7Hz, CH₂), 2.48-2.54 (2H, m, CH₂), 2.68 (2H, t, J 7.3Hz, tetrahydropyridinyl CH₂), 2.84-2.89 (2H, m, tetrahydropyridinyl CH₂), 3.66 (2H, s, NCH₂Ar), 5.34-5.38 (1H, m, tetrahydropyridinyl 5-H), 6.06 (1H, d, J 2.9Hz, furanyl 3-H), 6.31 (1H, dd, J 3.0, 1.9Hz, furanyl 4-H), 7.02 (1H, dd, J 7.8, 4.6Hz, 5-H), 7.32 (1H, d, J 2.3Hz, furanyl 5-H), 7.46 (1H, d, J 3.0Hz, 2-H), 7.99 (1H, m, dd, J 7.8, 4.1Hz, 4-H), 8.18 (1H, dd, J 4.6, 1.4Hz, 6-H), and 11.38 (1H, br s, NH); m/z (CI⁺, NH₃) 308 (M+1)⁺.

### EXAMPLE 19

### 3-(4-[2-(Tetrahydrofuran-2-yl)ethyl]-1,2,3,6-tetrahydropyridin-1-yl)methyl-1H-pyrrolo[2,3-b]pyridine

### Step 1: 4-(2-[Tetrahydrofuran-2-yl]ethyl)pyridine

A solution of 1-(pyridin-4-yl)-2-(furan-2-yl)ethene (15.0g, 87.7mmol) [Example 18, Step 1] in methanol (300ml) was treated with ammonium formate (27.6g, 438.5mmol) and 10% palladium on charcoal catalyst (1.5g, 10% (w/w)). The mixture was stirred at reflux for 3 hours. The catalyst was removed by filtration and the solvent was evaporated *in vacuo.* The residue was partitioned between dichloromethane and water. The organic phase was separated, dried (MgSO₄) and evaporated *in vacuo.* The residue was chromatographed with 1:1 hexane/ethyl acetate as eluant to afford the *title compound* as a colourless oil (3.0g, 19%); δ_{H} (DMSO-d₆) 1.35-1.50 (1H, m, tetrahydrofuranyl H), 1.70-2.00 (5H, m, CH₂ and 3 x tetrahydrofuranyl H), 2.54-2.76 (2H, m, CH₂), 3.54-3.82 (3H, m, tetrahydrofuranyl H), 7.22 (2H, d, J 7.1Hz, ArH), and 8.46 (2H, d J 7.0Hz, ArH); m/z (CI⁺, NH₃) 178 (M+1)⁺.

### Step 2: 1-Benzyl-4-(2-[tetrahydrofuran-2-yl]ethyl)-1,2,3,6-tetrahydropyridine

A solution of 4-(2-[tetrahydrofuran-2-yl]ethyl)pyridine (3.0g, 16.9mmol) in anhydrous dimethylformamide (5ml) was treated with benzyl bromide (2.2ml, 18.6mmol) and the mixture was stirred at room temperature for one hour. The reaction was diluted with absolute ethanol (100ml), treated with sodium borohydride (0.8g, 21.2mmol) and stirred at reflux for one hour. The solvent was evaporated *in vacuo* and the residue partitioned between diethyl ether and water. The organic layer was separated, dried (Na₂SO₄) and evaporated *in vacuo.* The residue was chromatographed with 3% methanol in dichloromethane as eluant to afford the *title compound* (3.75g, 82%) as a colourless oil; δ_{H} (DMSO-d₆) 1.30-2.08 (8H, m, 4 x CH₂), 2.45-2.52 (2H, m, tetrahydropyridinyl CH₂), 2.52-2.56 (2H, m, CH₂), 2.60-2.66 (2H, m, tetrahydropyridinyl CH₂), 3.50 (2H, s, PhCH₂N), 3.52-3.80 (3H, m, tetrahydrofuranyl CH₂ and CH), 5.50-5.55 (1H, m, tetrahydropyridinyl 5-H), and 7.20-7.34 (5H, m, ArH); m/z (CI⁺, NH₃) 272 (M+1)⁺.

### Step 3: 3-(4-[2-(Tetrahydrofuran-2-yl)ethyl]-1,2,3,6-tetrahydropyridin-1-yl)methyl-1H-pyrrolo[2,3-b]pyridine

A solution of the foregoing compound (3.75g, 13.8mmol) in anhydrous dichloromethane (150ml) was cooled to 0°C and treated with a solution of 1-chloroethylchloroformate (1.94ml, 18.0mmol) in anhydrous dichloromethane (50ml). After stirring at room temperature for one hour, the solvent was evaporated and the residue redissolved in methanol (200ml). This solution was stirred at reflux for two hours. The solvent was evaporated and the residue partitioned between dichloromethane and saturated aqueous potassium carbonate solution. The organic phase was separated, dried (MgSO₄) and evaporated to afford the crude product (2.49g, 99.6%) as a gummy solid.

A solution of the crude tetrahydropyridine (517mg, 2.86mmol) and 3-dimethylaminomethyl-1H-pyrrolo[2,3-b]pyridine (500mg, 2.86mmol) in toluene (10ml) was stirred at reflux for 10 hours. The solvent was evaporated and the residue chromatographed with 3% methanol in dichloromethane as eluant to afford the *title compound* (175mg, 20%) as a colourless solid, m.p. 70-72°C; (Found: C, 72.29; H, 7.86; N, 13.97. C₁₉H₂₅N₃O.0.25H₂O requires C, 72.23; H, 8.13; N, 13.30); δ_{H} (DMSO-d₆) 1.30-1.60 (3H, m, 3 x tetrahydrofuranyl H), 1.70-2.08 (7H, m, 1 x tetrahydrofuranyl H, CH₂CH₂ and 1 x tetrahydropyridinyl CH₂), 2.50 (2H, m, tetrahydropyridinyl CH₂ under DMSO peak), 2.86 (2H, br s, tetrahydropyridinyl CH₂), 3.50-3.76 (5H, m, ArCH₂N and 3 x tetrahydrofuranyl H), 5.30-5.36 (1H, m, tetrahydropyridinyl 5-H), 7.02 (1H, dd, J 7.8, 4.6Hz, 5-H), 7.33 (1H, d, J 2.2Hz, 2-H), 7.98 (1H, dd, J 7.8, 1.1Hz, 4-H), 8.18 (1H, dd, J 4.6, 1.1Hz, 6-H), and 11.42 (1H, m, NH). m/z (CI⁺, NH₃) 312 (M+1)⁺.

### EXAMPLE 20

### 3-(4-[2-(5-Methylfuran-2-yl)ethyl]-1,2,3,6-tetrahydropyridin-1-yl)methyl-1H-pyrrolo[2,3-b]pyridine

1-Benzyl-4-(2-[5-methylfuran-2-yl]ethyl)-1,2,3,6-tetrahydropyridine was prepared in the same manner as 1-benzyl-4-[2-furan-2-yl]ethyl)-1,2,3,6-tetrahydropyridine (Example 18).

A solution of 1-benzyl-4-(2-[5-methylfuran-2-yl]ethyl)-)-1,2,3,6-tetrahydropyridine (1.5g, 5.34mmol) in anhydrous dichloromethane (100ml) was cooled to 0°C and treated with a solution of 1-chloroethylchloroformate (0.75ml, 6.94mmol) in anhydrous dichloromethane (60ml). After stirring at room temperature for one hour, the solvent was evaporated and the residue redissolved in methanol (160ml). This solution was stirred at reflux for two hours. The solvent was evaporated and the residue partitioned between dichloromethane and saturated aqueous potassium carbonate solution. The organic phase was separated, dried (MgSO₄) and evaporated to afford the crude product (1.0g, 99%) as a brown oil.

A solution of the crude tetrahydropyridine (500mg, 2.6mmol) and 3-dimethylaminomethyl-1H-pyrrolo[2,3-b]pyridine (416mg, 2.38mmol) in toluene (20ml) was stirred at reflux for 8 hours. The solvent was evaporated and the residue chromatographed with 3% methanol in dichloromethane as eluant to afford the *title compound* (160mg, 21%) as a colourless solid, m.p. 118-119°C; (Found: C, 74.04; H, 7.01; N, 12.55. C₂₀H₂₃N₃O.0.175H₂O requires C, 74.01; H, 7.25; N, 12.95); δ_{H} (DMSO-d₆) 1.95-2.06 (2H, m, tetrahydropyridinyl CH₂), 2.14-2.24 (5H, m, CH₃ and CH₂CH₂), 2.48-2.54 (2H, m, CH₂CH₂), 2.61 (2H, t, J 7.4Hz, tetrahydropyridinyl CH₂), 2.84-2.93 (2H, m, tetrahydropyridinyl CH₂), 3.66 (2H, s, NCH₂Ar), 5.35-5.40 (1H, m, tetrahydropyridinyl 5-H), 5.87-5.95 (2H, m, furanyl H), 7.01 (1H, dd, J 7.9, 4.8Hz, 5H), 7.33 (1H, d, J 2.3Hz, 2-H), 7.99 (1H, dd, J 7.8, 1.2Hz, 4-H), 8.17 (1H, dd, J 4.6, 1.4Hz, 6-H), and 11.41 (1H, br s, NH); m/z (CI⁺, NH₃) 322 (M+1)⁺.

### EXAMPLE 21

### 3-(4-Ethyl-1,2,3,6-tetrahydropyridin-1-yl)methyl-1H-pyrrolo[2,3-b]pyridine

### Step 1: 1-Benzyl-4-ethyl-1,2,3,6-tetrahydropyridine

A solution of 4-ethylpyridine (3.0g, 28mmol) in anhydrous dimethylformamide (10ml) was treated with benzyl bromide (3.7ml, 31mmol) and the mixture was stirred at room temperature for two hours. The reaction mixture was diluted with absolute ethanol (60ml), treated with sodium borohydride (1.3g, 35mmol) and stirred at reflux for one hour. The solvent was evaporated *in vacuo* and the residue partitioned between dichloromethane and water. The organic phase was separated, dried (MgSO₄) and evaporated *in vacuo.* The residue was chromatographed with 1:1 hexane/ethyl acetate as eluant to give the *title compound* (1.92g, 34% over two steps) as a colourless oil; δ_{H} (CDCl₃) 1.00 (3H, t, J 7.5Hz, CH₂CH₃), 1.94-2.12 (4H, m, CH₂CH₃ and tetrahydropyridinyl CH₂), 2.55 (2H, t, J 6.3Hz, tetrahydropyridinyl CH₂), 2.90-3.00 (2H, m, tetrahydropyridinyl CH₂), 3.58 (2H, s, PhCH₂N), 5.32-5.40 (1H, m, tetrahydropyridinyl 5-H), and 7.20-7.40 (5H, m, ArH); m/z (CI⁺, NH₃) 202 (M+1)⁺.

### Step 2: 3-(4-Ethyl-1,2,3,6-tetrahydropyridin-1-yl)-methyl-1H-pyrrolo[2,3-b]pyridine

A solution of 1-benzyl-4-ethyl-1,2,3,6-tetrahydropyridine (1.92g, 9.6mmol) in anhydrous dichloromethane (50ml) was cooled to 0°C and treated with a solution of 1-chloroethylchloroformate (1.34ml, 12.4mmol) in anhydrous dichloromethane (10ml). After stirring at room temperature for one hour, the solvent was evaporated and the residue redissolved in methanol (50ml). This solution was heated at reflux for one hour. The solvent was evaporated and the residue partitioned between dichloromethane and saturated aqueous potassium carbonate solution. The organic phase was separated, dried (MgSO₄) and evaporated *in vacuo* to afford the crude product (320mg, 30%) as a colourless oil. A solution of the crude tetrahydropyridine (320mg, 2.9mmol) and 3-dimethylaminomethyl-1H-pyrrolo[2,3-b]pyridine (504mg, 2.9mmol) in toluene (10ml) was stirred at reflux for 16 hours. The solvent was evaporated and the residue chromatographed with 3% methanol in dichloromethane as eluant to afford the *title compound* (300mg, 43%) as a colourless solid, m.p. 88-90°C; (Found: C, 74.03; H, 7.94; N, 16.97. C₁₅H₁₉N₃.0.125H₂O requires C, 73.96; H, 7.97; N, 17.25%); δ_{H} (DMSO-d₆) 0.99 (3H, t, J 7.5Hz, CH₂CH₃), 1.97 (2H, q, J 7.5Hz, CH₂CH₃), 2.06-2.12 (2H, m, tetrahydropyridinyl CH₂), 2.61 (2H, t, J 5.8Hz, tetrahydropyridinyl CH₂), 3.02-3.08 (2H, m, tetrahydropyridinyl CH₂), 3.78 (2H, s, NCH₂Ar), 5.34-5.40 (1H, m, tetrahydropyridinyl 5-H), 7.08 (1H, dd, J 7.9, 4.8Hz, 5-H), 7.29 (1H, m, 2-H), 8.06-8.04 (1H, m, 4-H), 8.28-8.32 (1H, m, 6-H), 9.28 (1H, br s, NH). m/z (CI⁺, NH₃) 242 (M+1)⁺.

### EXAMPLE 22

### Benzoic acid 1-(1H)pyrrolo[2,3-b]pyridin-3-yl-methyl)-1,2,3,6-tetrahydropyridin-4-ylmethyl ester

### Step 1: 1-Benzyl-4-hydroxymethyl-1,2,3,6-tetrahydropyridine

A solution of 4-hydroxymethylpyridine (20g, 0.183mol) in anhydrous dimethylformamide (70ml) was treated with benzyl bromide (24ml, 0.202mol) and the mixture was stirred at 100°C for two hours. The reaction was cooled to room temperature and diluted with absolute ethanol (250ml). Sodium borohydride (8.7g, 0.229mol) was added portionwise and the mixture stirred at reflux for 3 hours. The solvent was evaporated *in vacuo* and the residue partitioned between dichloromethane and water. The organic phase was separated, dried (MgSO₄) and evaporated *in vacuo.* The residue was chromatographed with 1:1 hexane/ethyl acetate as eluant to the *title compound (13g,* 35%) as a lemon yellow solid; δ_{H} (CDCl₃) 2.12-2.20 (2H, m, tetrahydropyridinyl CH₂), 2.60 (2H, t, J 7.5Hz, tetrahydropyridinyl CH₂), 2.96-3.04 (2H, m, tetrahydropyridyl CH₂), 3.60 (2H, s, PhCH₂N), 4.00-4.04 (2H, m, CH₂OH), 5.60-5.66 (1H, m, tetrahydropyridinyl 5-H), and 7.20-7.40 (5H, m, ArH). m/z (CI⁺, NH₃) 204 (M+1)⁺.

### Step 2: Benzoic acid 1-benzyl-1,2,3,6-tetrahydropyridin-4-ylmethyl ester

A cooled (ice-bath) solution of the foregoing compound (lg, 4.9mmol) in dichloromethane (30ml) was treated with sodium hydroxide solution (2M, 30ml) and a solution of benzoylchloride (0.58ml, 4.9mmol) in dichloromethane (10ml). The mixture was stirred at 0°C for 2 hours. The organic layer was separated, dried (MgSO₄) and evaporated *in vacuo* to afford the *title compound* (1.37g, 91%) as a colourless oil;. δ_{H} (DMSO-d₆) 2.12.2.20 (2H, m, tetrahydropyridinyl CH₂), 2.55 (2H, t, J 7.5Hz, tetrahydropyridinyl CH₂), 2.95-3.05 (2H, m, tetrahydropyridinyl CH₂), 3.55 (2H, s, PhCH₂N), 4.72 (2H, s, CH₂O), 5.75-5.82 (1H, m, tetrahydropyridinyl 5-H), 7.22-7.38 (5H, m, ArH), 7.48-7.57 (2H, m, ArH), 7.64-7.70 (1H, m, ArH), and 7.96-8.04 (2H, m, ArH); m/z (CI⁺, NH₃) 308 (M+1)⁺.

### Step 3: Benzoic acid 1-(1H-pyrrolo[2,3-b]pyridin-3-ylmethyl)-1,2,3,6-tetrahydropyridin-4-ylmethyl ester

A solution of benzoic acid 1-benzyl-1,2,3,6-tetrahydropyridin-4-ylmethyl ester (1.35g, 4.4mmol) in anhydrous dichloromethane (50ml) was cooled to 0°C and treated with a solution of 1-chloroethyl-chloroformate (0.62ml, 5.7mmol) in anhydrous dichloromethane (10ml). After stirring at 0°C for 1.5 hours, the solvent was evaporated and the residue redissolved in methanol (50ml). This solution was stirred at reflux for two hours. The solvent was evaporated and the residue partitioned between ethyl acetate and saturated aqueous potassium carbonate solution. The organic phase was separated, dried (MgSO₄) and evaporated *in vacuo* to afford the crude product (520mg, 58% over two steps) as a colourless oil. A solution of the crude tetrahydropyridine (520mg, 2.5mmol) and 3-dimethylaminomethyl-1H-pyrrolo[2,3-b]pyridine (520mg, 2.5mmol) in toluene (20ml) was stirred at reflux for 9 hours. The solvent was evaporated and the residue chromatographed with 5% methanol in dichloromethane as eluant to afford the *title compound* (480mg, 55%) as a colourless solid, m.p. 126°C; (Found: C, 72.42; H, 6.08; N, 12.15. C₂₁H₂₁N₃O₂ requires C, 72.60; H, 6.09; N, 12.10%); δ_{H} (CDCl₃) 2.20-2.30 (2H, m, tetrahydropyridinyl CH₂), 2.68 (2H, t, J 5.7Hz, tetrahydropyridinyl CH₂), 3.05-3.10 (2H, m, tetrahydropyridinyl CH₂), 3.80 (2H, s, NCH₂Ar), 5.86-5.94 (1H, m, tetrahydropyridinyl 5-H), 4.74 (2H, s, CH₂O), 7.07 (1H, dd, J 7.8, 4.7Hz, 5-H), 7.20-7.30 (1H, m, ArH), 7.38-7.45 (2H, m, ArH), 7.50-7.60 (1H, m, ArH), 8.00-8.10 (3H, m, ArH), 8.30 (1H, dd, J 4.7, 1.5Hz, 6-H), and 9.33 (1H, s, NH); m/z (CI⁺, NH₃) 348 (M+1)⁺.

### EXAMPLE 23

### (E)-3-(4-[2-(3-Methoxyphenyl)ethenyl]-1,2,3,6-tetrahydropyridin-1-yl)methyl-1H-pyrrolo[2,3-b]pyridine

M.p. 186-188°C (PhMe); (Found: C, 76.52; H, 6.93; N, 11.55. C₂₂H₂₃N₃O requires C, 76.49; H, 6.71; N, 12.16%); δ_{H} (CDCl₃) 2.44 (2H, br s, tetrahydropyridinyl CH₂), 2.77 (2H, br s, tetrahydropyridinyl CH₂), 3.23 (2H, br s, tetrahydropyridinyl CH₂), 3.81 (3H, s, OCH₃), 3.86 (2H, s, ArCH₂N), 5.81 (1H, br s, tetrahydropyridinyl 5-H), 6.41 (1H, d, J 16.0Hz, ArCH=CHR), 6.75-6.79 (1H, m, 4'-H), 6.77 (1H, d, J 16.0Hz, ArCH=CHR), 6.93 (1H, br s, 2'-H), 6.99 (1H, d, J 7.7Hz, 6'-H), 7.08 (1H, dd, J 7.9, 4.8Hz, 5-H), 7.22 (1H, t, J 7.9Hz, 5'-H), 7.33 (1H, br s, 2-H), 8.09 (1H, d, J 7.9Hz, 4-H), 8.31 (1H, dd, J 4.8, 1.6Hz, 6-H), and 9.15 (1H, br s, NH); m/z (CI⁺, NH₃) 346 (M+1)⁺.

### EXAMPLE 24

### 3-(4-Phenoxymethyl-1,2,3,6-tetrahydropyridin-1-yl)methyl-1H-pyrrolo[2,3-b]pyridine

M.p. 161-163°C (MeOH); (Found: C, 75.05; H, 6.61; N, 12.91. C₂₀H₂₁N₃O requires C, 75.21; H, 6.63; N, 13.16%); δ_{H} (CDCl₃) 2.25 (2H, br s, tetrahydropyridinyl CH₂), 2.69 (2H, t, J 5.7Hz, tetrahydropyridinyl CH₂), 3.09(2H, br s, tetrahydropyridinyl CH₂), 3.81 (2H, s, ArCH₂N), 4.40 (2H, s, ArCH₂O), 5.77 (1H, br s, CH=CR), 6.89-6.95 (3H, m, ArH), 7.08 (1H, dd, J 7.8, 4.7Hz, 5-H), 7.24-7.29 (3H, m, ArH), 8.07 (1H, dd, J 7.9, 1.5Hz, 4-H), 8.31 (1H, dd, J 4.8, 1.6Hz, 6-H), and 9.69 (1H, br s, NH); m/z (CI⁺, NH₃) 320 (M+1)⁺.

### EXAMPLE 25

### (E)-3-(4-[2-(5-Methylfuran-2-yl)ethenyl]-1,2,3,6-tetrahydropyridin-1-yl)methyl-1H-pyrrolo[2,3-b]pyridine

### Step 1: (E)-1-tert-Butyloxycarbonyl-3-(4-[2-(5-methylfuran-2-yl)ethenyl]-1,2,3,6-tetrahydropyridin-1-yl)methylpyrrolo[2,3-b]pyridine

A solution of 1-(5-methylfuran-2-yl)-2-(pyridin-4-yl)ethene (315mg, 1.7mmol) in anhydrous dimethylformamide (5ml) was treated with a solution of (*E*)-1-*tert*-butyloxycarbonyl-3-chloromethylpyrrolo[2,3-b]pyridine (500mg, 1.9mmol) [prepared using the method of Sanchez-Obregon *et al.,* Can. J. Chem., 1992, 70, 1531-1536] in anhydrous dimethylformamide (5ml). The mixture was stirred at 70°C for 16 hours. The reaction was diluted with ethanol (50ml) and treated with sodium borohydride (81mg, 2.13mmol). The mixture was stirred at room temperature for a further two hours whereupon the solvent was evaporated *in vacuo* and the residue partitioned between ethyl acetate and saturated brine solution. The organic layer was separated, dried (Na₂SO₄) and evaporated *in vacuo.* The residue was chromatographed on silica gel with 50% hexane/ethyl acetate as eluant to afford the *title compound* as an orange oil (160mg, 22%); δ_{H} (CDCl₃) 1.60 (9H, s, C(CH₃)₃), 2.20-2.33 (5H, m, tetrahydropyridinyl CH₂ and ArCH₃), 2.58-2.66 (2H, m, tetrahydropyridinyl CH₂), 3.06-3.16 (2H, m, tetrahydropyridinyl CH₂), 3.67 (2H, 2, NCH₂Ar), 5.67-5.75 (1H, m, tetrahydropyridinyl 5-H), 5.87-5.91 (1H, m, furan 4-H), 6.04-6.07 (1H, m, furan 3-H), 6.13 (1H, d, J 16.2Hz, CH=CHAr), 6.58 (1H, d, J 16.1Hz, CH=CHAr), 7.08-7.14 (1H, m, 5-H), 7.48-7.52 (1H, m, 2-H), 7.96-8.04 (1H, m, 4-H), and 8.40-8.46 (1H, m, 6-H); m/z (CI⁺, NH₃) 420 (M+1)⁺.

### Step 2: (E)-3-(4-[2-(5-methylfuran-2-yl)ethenyl]-1,2,3,6-tetrahydropyridin-1-yl)methyl-1H-pyrrolo[2,3-b]pyridine

A solution of (*E*)-1-*tert*-butyloxycarbonyl-3-(4-[2-(5-methylfuran-2-yl)ethenyl]-1,2,3,6-tetrahydropyridin-1-yl)methylpyrrolo[2,3-b]pyridine (160mg, 0.38mmol) in anhydrous dichloromethane (5ml) was treated with trifluoroacetic acid (500ml, 6.5mmol) and stirred at room temperature for two hours. Ammonium hydroxide solution (5ml) was added and the whole mixture evaporated *in vacuo.* The residue was azeotroped with toluene. The crude material was chromatographed on silica gel with 5% methanol in dichloromethane as eluant to afford the *title compound* as a pale lemon solid (70mg, 57%), m.p. 180°C (dec.); (Found: C, 71.47; H, 6.46; N, 12.01. C₂₀H₂₁N₃O.H₂O requires C, 71.19; H, 6.87; N, 12.42%); δ_{H} (DMSO-d₆) 2.15-2.30 (5H, m, tetrahydropyridinyl CH₂ and ArCH₃), 2.59 (2H, t, J 5.5Hz, tetrahydropyridinyl CH₂), 3.02-3.10 (2H, m, tetrahydropyridinyl CH₂), 3.71 (2H, s, NCH₂Ar), 5.78-5.86 (1H, m, tetrahydropyridinyl 5-H), 6.05-6.10 (1H, m, furan 4-H), 6.16-6.30 (2H, m, CH=CHAr and furan 3-H), 6.55 (1H, d, J 16.1Hz, CH=CHAr), 7.02 (1H, dd, J 7.8, 4.8Hz, 5-H), 7.34-7.38 (1H, m, 2-H), 8.01 (1H, d, J 7.8Hz, 4-H), 8.18 (1H, d, J 4.6Hz, 6-H), and 11.45 (1H, s, NH); m/z (CI⁺, NH₃) 320 (M+1)⁺.

### EXAMPLE 26

### 6-Phenyl-2-(1H-pyrrolo[2,3-b]pyridin-3-ylmethyl)-1,2,3,4-tetrahydroisoquinoline

A mixture of 6-phenyl-1,2,3,4-tetrahydroisoquinoline (prepared by the method of L N Pridgen, *J. Heterocyclic Chem.* 1980, **17**, 1289) (0.5g, 2.38mmol) and 3-dimethylaminomethyl-1H-pyrrolo[2,3-b]pyridine (0.4g, 2.28mmol) in toluene (5ml) was heated at reflux under nitrogen for 18h. The mixture was allowed to cool and the crystallised product collected. Recrystallisation from ethyl acetate afforded the *title compound* (0.13g, 17%), m.p. 211-213°C; (Found: C, 80.15; H, 6.30; N, 12.32. C₂₃H₂₁N₃.0.3H₂O requires C, 80.11; H, 6.31; N, 12.18%); δ_{H} (DMSO-d₆) 2.73 (2H, br s, CH₂CH₂Ph), 2.87 (2H, br s, NCH₂CH₂), 3.61 (2H, s, ArCH₂N), 3.83 (2H, s, NCH₂Ph), 7.02 (1H, dd, J 7.8, 4.6Hz, 5-H), 7.18 (1H, d, J 3.2Hz, 2-H), 7.30-7.45 (6H, m, ArH), 7.60 (2H, d, J 7.6Hz, ArH), 8.05 (1H, d, J 7.6Hz, 4-H), 8.20 (1H, dd, J 6.1, 1.4Hz, 6-H), and 11.48 (1H, br s, NH); m/z (CI⁺, NH₃) 340 (M+1)⁺.

### EXAMPLE 27

### 6-Methoxy-2-(1H-pyrrolo[2,3-b]pyridin-3-ylmethyl)-1,2,3,4-tetrahydroisoquinoline

A mixture of 6-methoxy-1,2,3,4-tetrahydroisoquinoline (prepared by the method of Helfer, *Helv. Chim. Acta,* 1924, **7**, 945) (0.55g, 3.36mmol) and 3-dimethylaminomethyl-1H-pyrrolo[2,3-b]pyridine (0.59g, 3.36mmol) in toluene (3ml) was heated at reflux under nitrogen for 18h. The mixture was allowed to cool and the crystallised product collected. Recrystallisation from toluene afforded the *title compound* (0.31g, 32%), m.p. 144-146°C; (Found: C, 73.04; H, 6.43; N, 14.10. C₁₈H₁₉N₃O.0.1H₂O requires C, 73.24; H, 6.55; N, 14.23%); δ_{H} (DMSO-d₆) 2.67 (2H, br s, CH₂), 2.76 (2H, t, J 5.4Hz, CH₂), 3.49 (2H, s, CH₂), 3.69 (3H, s, OCH₃), 3.78 (2H, s, CH₂), 6.65 (2H, m, ArH), 6.89 (1H, d, 9Hz, ArH), 7.01 (1H, dd, J 7.9, 4.7Hz, 5-H), 7.40 (1H, d, J 2.2Hz, ArH), 8.03 (1H, dd, J 7.7, 1.3Hz, 4-H), 8.19 (1H, dd, J 4.6, 1.4Hz, 6-H), and 11.47 (1H, br s, NH); m/z (CI⁺, NH₃) 294 (M+1)⁺.

### EXAMPLE 28

### 7-Phenyl-2-(1H-pyrrolo[2,3-b]pyridin-3-ylmethyl)-1,2,3,4-tetrahydroisoquinoline

### Step 1: 7-Phenylisoquinoline

To a slurry of 7-(trifluoromethanesulfonyloxy)isoquinoline (prepared by the method of D.F. Ortwine *et al., J. Med. Chem.,* 1992, **35**, 1345) (2.lg, 7.5mmol) in toluene (20ml) was added phenyl boronic acid (1.21g, 10mmol) and a 2M solution of sodium carbonate (15ml). The reaction vessel was filled with nitrogen, tetrakis(triphenylphosphine)palladium(0) (0.25g, 0.22mmol) was added and the reaction mixture was heated at 90°C for 18h. The reaction mixture was diluted with ethyl acetate (50ml) and washed with sodium carbonate solution (50ml). The aqueous solution was extracted with ethyl acetate (2 x 50ml). The ethyl acetate extracts were combined, dried over magnesium sulphate, filtered and evaporated under reduced pressure to give an oil. The oil was purified by flash chromatography using hexane/ethyl acetate (1:1) as eluant to give the *title compound* (1.27g, 83%); δ_{H} (DMSO-d₆) 7.41-7.88 (6H, m, ArH and isoquinolinyl H), 7.95 (1H, d, J 6.2Hz, isoquinolinyl H), 8.14 (1H, dd, J 8.5, 1.75Hz, isoquinolinyl H), 8.44 (1H, t, J 0.85Hz, isoquinolinyl H), 8.52 (1H, d, 5.75Hz, 3-H), and 9.31 (1H, s, 1-H); m/z (CI⁺, NH₃) 206 (M+1)⁺.

### Step 2: 7-Phenyl-1,2,3,4-tetrahydroisoquinoline

To a solution of 7-phenylisoquinoline (1.25g, 6.lmmol) in methanol (50ml) was added conc HCl (2ml) and platinum oxide (100mg). The reaction mixture was hydrogenated at 50 psi until no further uptake of hydrogen was observed. The catalyst was filtered off, washed with methanol. The filtrate was evaporated under reduced pressure to give a solid. The solid was partitioned between ethyl acetate and sodium carbonate solution. The aqueous solution was extracted with ethyl acetate (2 x 100ml). The ethyl acetate extracts were combined, washed with brine (2 x 100ml), dried over magnesium sulphate, filtered and evaporated under reduced pressure to give an oil, which crystallised on standing. The solid was a mixture of the *title compound* and 7-phenyl-decahydroisoquinoline; Rf 0.66 (EtOAc/MeOH/NH₃ 5:1:1).

### Step 3: 7-Phenyl-2-(1H-pyrrolo[2,3-b]pyridin-3-ylmethyl)-1,2,3,4-tetrahydroisoquinoline

The mixture obtained from the previous step (0.5g, 2.38mmol) and 3-dimethylaminomethyl-1H-pyrrolo[2,3-b]pyridine (0.42g, 2.39mmol) in toluene (3ml) was heated at reflux under nitrogen for 24h. The mixture was allowed to cool and the crystalline product collected. The solid was purified by flash chromatography, eluting with dichloromethane/methanol/ammonia. The apropriate fractions were combined to give a solid (0.54g). The solid was recrystallised from toluene, and purified by reverse phase HPLC on a KR100, 5µm C₈ column (250mm x 20mm id) using 40% acetonitrile, 60% H₂O (containing 0.1% TFA) as eluant. The appropriate fraction was evaporated under reduced pressure to dryness. The residue was partitioned between dichloromethane/methanol (9:1) and sodium hydroxide solution. The organic phase was evaporated under reduced pressure to give a solid. The solid was recrystallised from toluene to give the *title compound* (0.14g, 17%), m.p. 203-205°C; (Found: C, 79.92; H, 6.02; N, 12.10. C₂₃H₂₁N₃.0.35H₂O requires C, 79.90; H, 6.33; N, 12.15%); δ_{H} (DMSO-d₆) 2.72 (2H, t, J 5.4Hz, CH₂), 2.82 (2H, t, J 5.4Hz, CH₂), 3.63 (2H, s, CH₂), 3.82 (2H, s, CH₂), 7.02 (1H, dd, J 7.6, 4.7Hz, 5-H), 7.16 (1H, d, J 7.9Hz, ArH), 7.29-7.43 (6H, m, ArH), 7.59 (2H, dd, J 6.8, 1.4Hz, ArH), 8.05 (1H, d, J 7.9Hz, 4-H), 8.20 (1H, dd, J 4.7, 1.4Hz, 6-H), and 11.49 (1H, br s, NH); m/z (CI⁺, NH₃) 340 (M+1)⁺.

### EXAMPLE 29

### 7-Benzyloxy-2-(1H-pyrrolo[2,3-b]pyridin-3-ylmethyl)-1,2,3,4-tetrahydroisoquinoline

### Step 1: 7-Benzyloxyisoquinoline

To a solution of 7-hydroxyisoquinoline (prepared by the method of R.B. Woodward and W.D. Doering, *J. Am. Chem. Soc.,* 1945, **67**, 860) (1.45g, 10mmol) in DMF (20ml) was added sodium hydride (60% dispersion in oil, 0.4g, 10mmol) portionwise. After stirring for 30 minutes, benzyl bromide (1.2ml, 10mmol) was added and the reaction mixture stirred for 2h. The reaction mixture was poured into water and extracted with diethyl ether (3 x 100ml). The ether extracts were combined, washed with 2N sodium hydroxide solution (2 x 50ml), brine (50ml), dried over magnesium sulphate, filtered and evaporated under reduced pressure to give the *title compound* (1.6g, 68%); δ_{H} (CDCl₃) 5.21 (2H, s, OCH₂), 7.30-7.51 (7H, m, ArH), 7.59 (1H, d, J 8.3Hz, ArH), 7.75 (1H, d, J 13Hz, ArH), 8.42 (1H, d, J 8Hz, ArH), and 9.14 (1H, s, ArH).

### Step 2: 7-Benzyloxy-1,2,3,4-tetrahydroisoquinoline

7-Benzyloxyisoquinoline (1.5g, 6.37mmol) in methanol (100ml) was hydrogenated at 50 psi on a Parr apparatus using platinum oxide (100mg) as catalyst. The catalyst was collected by filtration, the filtrate evaporated under reduced pressure to give an oil which crystallised on treatment with hexane/diethyl ether to give the *title compound* (0.96g, 64%); δ_{H} (CDCl₃) 2.72 (2H, t, J 6Hz, CH₂), 3.11 (2H, t, J 6Hz, CH₂), 3.90 (2H, s, CH₂), 6.62 (1H, d, J 2.25Hz, tetrahydroisoquinolinyl H), 6.78 (1H, dd, J 5.75, 2.75Hz, tetrahydroisoquinolinyl H), 6.99 (1H, d, J 9.25Hz, tetrahydroisoquinolinyl H), and 7.25-7.43 (5H, m, ArH).

### Step 3: 7-Benzyloxy-2-(1H-pyrrolo[2,3-b]pyridin-3-ylmethyl)-1,2,3,4-tetrahydroisoquinoline

A mixture of 7-benzyloxy-1,2,3,4-tetrahydroisoquinoline (0.48g, 2mmol) and 3-dimethylaminomethyl-1H-pyrrolo[2,3-b]pyridine (0.35g, 2mmol) in toluene (5ml) was heated at reflux under nitrogen for 18h. The mixture was allowed to cool and the crystallised product collected. The product was purified by flash chromatography using dichloromethane/methanol (10:1) as eluant. The appropriate fractions were combined and evaporated to give a solid. Recrystallisation from toluene afforded the *title compound* (0.24g, 32%) m.p. 180-182°C; (Found: C, 77.73; H, 6.00; N, 11.11. C₂₄H₂₃N₃O requires C, 78.02; H, 6.27; N, 11.37%); δ_{H} (DMSO-d₆) 2.68 (4H, m, 2 x CH₂), 3.51 (2H, s, CH₂), 3.78 (2H, s, CH₂), 5.01 (2H, s, OCH₂), 6.66 (1H, d, J 2.4Hz, ArH), 6.74 (1H, dd, J 8.3, 2.6Hz, ArH), 7.02 (2H, m, ArH), 7.32-7.42 (6H, m, ArH), 8.03 (1H, dd, J 7.9, 1.2Hz, 4-H), 8.18 (1H, dd, J 4.7, 1.5Hz, 6-H), and 11.47 (1H, br s, NH); m/z (CI⁺, NH₃) 370 (M+1)⁺.

### EXAMPLE 30

### 7-(1H-Pyrrolo[2,3-b]pyridin-3-ylmethyl)-7-thiophen-3-yl-1,2,3,4-tetrahydroisoquinoline

### Step 1: 7-(3-Thienyl)isoquinoline

Using the method described in Example 28, Step 1 and 3-thiophene boronic acid (1g, 8mmol) the *title compound was* obtained (0.7g, 54%); δ_{H} (CDCl₃) 7.47 (1H, dd, J 5.1, 2.9Hz, thiophene 5-H), 7.53 (1H, dd, J 5.1, 1.3Hz, thiophene 4-H), 7.61 (1H, dd, J 2.9, 1.3Hz, thiophene 2-H), 7.64 (1H, d, J 5.7Hz, isoquinolinyl H), 7.85 (1H, d, J 8.5Hz, isoquinolinyl H), 7.96 (1H, m, isoquinolinyl H), 8.14 (1H, s, isoquinolinyl H), 8.52 (1H, d, J 5.7Hz, isoquinolinyl 3-H), and 9.28 (1H, s, isoquinolinyl 1-H); m/z (CI⁺, NH₃) 212 (M+1)⁺.

### Step 2: 2-(1H-Pyrrolo[2,3-b]pyridin-3-ylmethyl)-7-thiophen-3-yl-1,2,3,4-tetrahydroisoquinoline

A solution of 7-(3-thienyl)isoquinoline (0.15g, 3mmol) in dichloromethane was treated with an excess of HCl gas. The mixture was evaporated under reduced pressure to give a colourless solid. The solid was dissolved in ethanol, platinum oxide (100mg) added, the reaction mixture hydrogenated at 50 psi on a Parr apparatus until uptake of hydrogen ceased. The catalyst was collected by filtration and the filtrate was hydrogenated again using fresh catalyst until the starting material had been consumed. The filtrate was evaporated under reduced pressure. The residue was dissolved in ethyl acetate (100ml), washed with sodium carbonate solution (100ml), dried over magnesium sulphate, filtered and evaporated under reduced pressure to give crude 7-(3-thienyl)-1,2,3,4-tetrahydroisoquinoline as an oil (0.41g, 62%). A mixture of this oil (0.40g, 1.85mmol) and 3-dimethylaminomethyl-1H-pyrrolo[2,3-b]pyridine (0.325g, 1.85mmol) in toluene (3ml) was heated at reflux under nitrogen for 6h. The mixture was allowed to cool and the crystallised product collected. Recrystallisation from toluene afforded the *title compound* (0.17g, 28%), m.p. 228-230°C; (Found: C, 72.82; H, 5.46; N, 12.05. C₂₁H₁₉N₃S requires C, 73.01; H, 5.54; N, 12.16%); δ_{H} (DMSO-d₆) 2.72 (2H, t, J 5.4Hz, CH₂), 2.80 (2H, t, J 5Hz, CH₂), 3.59 (2H, s, CH₂), 3.82 (2H, s, CH₂), 7.02 (1H, dd, J 7.7, 4.7Hz, 5-H), 7.11 (1H, d, J 8.0Hz, ArH), 7.35 (1H, s, ArH), 7.42-7.50 (3H, m, ArH), 7.57 (1H, dd, J 5.1, 3Hz, ArH), 7.76 (1H, s, ArH), 8.04 (1H, dd, J 7.7, 1Hz, 4-H), 8.20 (1H, dd, J 4.6, 1Hz, 6-H), and 11.49 (1H, br s, NH); m/z (CI⁺, NH₃) 345 (M+1)⁺.

### EXAMPLE 31

### 6-(4-Chlorophenyl)-2-(1H-pyrrolo[2,3-b]pyridin-3-ylmethyl)-1,2,3,4-tetrahydroisoquinoline

### Step 1: 6-Hydroxy-3,4-dihydro-1H-isoquinoline-2-carboxylic acid tert-butyl ester

To a slurry of 6-hydroxy-1,2,3,4-tetrahydroisoquinoline hydrobromide (prepared by the method of Grimewald, *J. Med. Chem.,* 1987, **30**, 2208) (1.2g, 5.2mmol) and triethylamine (0.8ml, 5.7mmol) in dichloromethane (40ml) was added a solution of di-*tert*-butyl dicarbonate (1.2g, 5.5mmol) in dichloromethane (10ml). The mixture was stirred for 2h. The reaction mixture was washed with a solution of citric acid (2 x 50ml), brine (2 x 50ml), dried over magnesium sulphate, filtered and evaporated under reduced pressure to give the *title compound* as an oil (1.3g, 100%); δ_{H} (CDCl₃) 1.44 (9H, s, C(CH₃)₃), 2.77 (2H, t, J 6Hz, CH₂), 3.79 (2H, br s, CH₂), 4.54 (2H, s, CH₂), 6.64 (1H, s, ArH), 6.69 (1H, d, J 7.5Hz, ArH), and 6.96 (1H, d, J 8.5Hz, ArH).

### Step 2: 6-Trifluoromethanesulfonyloxy-3,4-dihydro-1H-isoquinoline-2-carboxylic acid tert-butyl ester

To a solution of 6-hydroxy-3,4-dihydro-1H-isoquinoline-2-carboxylic acid *tert*-butyl ester (1.3g, 5.2mM) and diisopropyethylamine (1ml) in methanol (40ml) at 0°C was added a solution of N-phenyltrifluoromethanesulfonimide (2.14g, 6mmol). On completion of the addition the reaction mixture was allowed to warm to room temperature and stirred overnight. A further equivalent of diisopropylethylamine (1ml) was added, the reaction mixture cooled to 0°C then a solution of N-phenyltrifluoromethanesulfonimide (2.14g, 6mmol) was added and the reaction mixture stirred at room temperature for a further 18h. The solvent was evaporated under reduced pressure and the residue purified by flash chromatography using hexane/ethyl acetate (4:1) as eluant. The *title compound* was obtained as an oil, which was taken on crude to the next step without further purification.

### Step 3: 6-(4-Chlorophenyl)-3,4-dihydro-1H-isoquinoline-2-carboxylic acid tert-butyl ester

To a mixture of 6-trifluoromethanesulfonyloxy-3,4-dihydro-1H-isoquinoline-2-carboxylic acid tert-butyl ester (2g, 5.25mmol) and 4-chlorophenyl boronic acid (1.17g, 7.5mmol) in toluene (20ml) was added a 2M sodium carbonate solution (7.5ml). The reaction mixture was stirred under a nitrogen atmosphere, then tetrakis(triphenylphosphine)palladium(0) (0.29g, 0.25mmol) was added and the reaction mixture was heated at 90°C for 18h. After allowing to cool the reaction mixture was diluted with water (100ml) and extracted with ethyl acetate (3 x 100ml). The ethyl acetate extracts were combined, dried over magnesium sulphate, filtered and evaporated under reduced pressure to give an oil. Purification by flash chromatography using hexane/ethyl acetate as eluant gave the *title compound* as an oil (0.7g, 39%); δ_{H} (CDCl₃) 1.5 (9H, s, C(CH₃)₃), 2.89 (2H, t, J 8.3Hz, CH₂), 3.68 (2H, t, J 8.3Hz, CH₂), 4.61 (2H, s, CH₂), and 7.15-7.5 (7H, m, ArH).

### Step 4: 6-(4-Chlorophenyl)-1,2,3,4-tetrahydroisoquinoline

To a solution of 6-(4-chlorophenyl)-3,4-dihydro-1H-isoquinoline-2-carboxylic acid *tert*-butyl ester (0.5g, 1.45mmol) in dichloromethane (6ml) was added trifluoroacetic acid (3ml). The reaction mixture was stirred at room temperature for 1h. The solvent was evaporated under reduced pressure and the residue partitioned between dichloromethane (3 x 20ml) and 1N sodium hydroxide solution (30ml). The combined organic phases were dried over potassium carbonate, filtered and evaporated under reduced pressure to give an oil. The oil was purified by flash chromatography using dichloromethane/methanol/ammonia 95:5:1 as eluant to afford the *title compound* as a solid (0.27g, 76%); δ_{H} (CDCl₃) 2.9 (2H, t, CH₂), 3.24 (2H, t, CH₂), 4.1 (2H, s, CH₂), and 7.04-7.55 (7H, m, ArH).

### Step 5: 6-(4-Chlorophenyl)-2-(1H-pyrrolo[2,3-b]pyridin-3-ylmethyl)-1,2,3,4-tetrahydroisoquinoline

A mixture of 6-(4-chlorophenyl-1,2,3,4-tetrahydroisoquinoline (0.23g, 0.94mmol) and 3-dimethylaminomethyl-1H-pyrrolo[2,3-b]pyridine (0.168g, 0.96mmol) in toluene (15ml) was heated at reflux under nitrogen for 18h. The mixture was allowed to cool and the crystallised product collected. Trituration with ethanol gave the *title compound* (0.18g, 48%), m.p. 202-204°C; (Found: C, 73.02; H, 5.20; N, 11.04. C₂₃H₂₀ClN₃.0.2H₂O requires C, 73.18; H, 5.45; N, 11.18%); δ_{H} (CDCl₃) 2.83 (2H, t, J 5.5Hz, CH₂), 2.94 (2H, t, J 5.5Hz, CH₂), 3.73 (2H, s, CH₂), 3.91 (2H, s, CH₂), 7.04-7.10 (2H, m, ArH), 7.29-7.50 (7H, m, ArH), 8.12 (7H, dd, J 7.8, 1.5Hz, 4-H), 8.32 (1H, dd, J 4.75, 1.5Hz, 6-H), and 9.60 (1H, br s, NH); m/z (CI⁺, NH₃) 374 (M+1)⁺.

### EXAMPLE 32

### 5-Phenyl-2-(1H-pyrrolo[2,3-b]pyridin-3-ylmethyl)-1,2,3,4-tetrahydroisoquinoline

Using the procedure described for Example 26 replacing 6-phenyl-1,2,3,4-tetrahydroisoquinoline with 5-phenyl-1,2,3,4-tetrahydroisoquinoline (prepared by the method of L.N. Pridgen, *J. Heterocyclic Chem.,* 1980, **17**, 1289) the *title compound* was obtained as a colourless solid, m.p. 202-204°C (toluene); (Found: C, 81.05; H, 6.31; N, 11.90. C₂₃H₂₁N₃.0.1C₇H₈.0.1H₂O requires C, 81.23; H, 6.33; N, 11.99%); δ_{H} (DMSO-d₆) 2.69 (4H, br s, NCH₂CH₂Ar), 3.45 (2H, s, ArCH₂N), 3.78 (2H, s, ArCH₂N), 5.08 (2H, s, ArCH₂O), 6.60 (1H, d, J 8Hz, 6'-H), 6.81 (1H, d, J 8Hz, 8'-H), 6.95-7.05 (2H, m, ArH), 7.3-7.5 (6H, m, ArH), 8.02 (1H, d, J 8Hz, 4-H), 8.20 (1H, br s, 6-H), and 11.5 (1H, br s, NH); m/z (CI⁺, NH₃) 340 (M+1)⁺.

### EXAMPLE 33

### 5-Benzyloxy-2-(1H-pyrrolo[2,3-b]pyridin-3-ylmethyl)-1,2,3,4-tetrahydroisoquinoline

Following the procedure for the preparation of Example 29, replacing 7-hydroxyisoquinoline in Step 1 with commercially available 5-hydroxyisoquinoline, afforded the *title compound* as a colourless solid, m.p. 179-181°C (MeOH/EtOH); (Found: C, 77.54; H, 6.19; N, 11.09. C₂₄H₂₃N₃O.0.1H₂O requires C, 77.54; H, 6.19; N, 11.09%); δ_{H} (DMSO-d₆) 2.69 (4H, br s, NCH₂CH₂Ar), 3.45 (2H, s, ArCH₂N), 3.78 (2H, s, ArCH₂N), 5.08 (2H, s, ArCH₂O), 6.60 (1H, d, J 8Hz, 6'-H), 6.81 (1H, d, J 8Hz, 8'-H), 6.95-7.05 (2H, m, ArH), 7.3-7.5 (6H, m, ArH), 8.02 (1H, d, J 8Hz, 4-H), 8.20 (1H, br s, 6-H), and 11.5 (1H, br s, NH); m/z (CI⁺, NH₃) 370 (M+1)⁺.

### EXAMPLE 34

### 5-Phenoxy-2-(1H-pyrrolo[2,3-b]pyridin-3-ylmethyl)-1,2,3,4-tetrahydroisoquinoline

### Step 1: 5-Phenoxyisoquinoline

Cupric oxide (0.2g) was added in one portion to a mixture of phenol (2g, 21mmol), potassium carbonate (2.7g, 20mmol), and 5-bromoisoquinoline (2.1g, 10mmol) in pyridine (20ml) heated at 90°C under nitrogen. The mixture was then heated at 140°C for 18h. After this time the pyridine was removed *in vacuo,* the residue suspended in ether and the mixture filtered through a pad of silica gel. The solvent was evaporated and the residue purified by column chromatography on silica with ethyl acetate/hexane (1:3-1:1) as eluant to give the *title compound* as a colourless solid (1.1g, 49%); δ_{H} (CDCl₃) 7.1-7.5 (7H, m, ArH), 7.75 (1H, d, J 8Hz, ArH), 8.02 (1H, d, J 8Hz, ArH), 8.57 (1H, d, J 8Hz, ArH), and 9.3 (1H, s, 1-H).

### Step 2: 5-Phenoxy-1,2,3,4-tetrahydroisoquinoline

A solution of 5-phenoxyisoquinoline (1g, 4.5mmol) in methanol (50ml) was shaken on a Parr hydrogenator at 55 psi hydrogen in the presence of platinum oxide (0.2g) for 3h. The catalyst was then removed by filtration and the solvent evaporated. The residue was triturated with hexane to give the *title compound* as a colourless solid (0.6g, 60%); δ_{H} (CDCl₃) 2.65-2.75 (2H, m, ArCH₂CH₂N), 3.1-3.2 (2H, m, ArCH₂CH₂N), 4.05 (2H, br s, ArCH₂N), 6.75 (1H, d, J 8Hz, ArH), 6.83 (1H, d, J 8Hz, ArH), and 6.9-7.35 (6H, m, ArH); m/z (CI⁺, NH₃) 226 (M+1)⁺.

### Step 3: 5-Phenoxy-2-(1H-pyrrolo[2,3-b]pyridin-3-ylmethyl)-1,2,3,4-tetrahydroisoquinoline

Following the procedure described for Example 26 replacing 6-phenyl-1,2,3,4-tetrahydroisoquinoline with 5-phenoxy-1,2,3,4-tetrahydroisoquinoline the *title compound* was obtained as a colourless solid, m.p. 179-180°C (EtOH/MeOH); (Found: C, 77.55; H, 5.96; N, 11.68. C₂₃H₂₀N₃O requires C, 77.94; H, 5.69; N, 11.86%); δ_{H} (CDCl₃) 2.8 (4H, m, ArCH₂CH₂N), 3.71 (2H, s, ArCH₂N), 3.89 (2H, s, ArCH₂N), 6.72 (1H, d, J 8Hz, ArH), 6.83 (1H, d, J 8Hz, ArH), 6.9-7.35 (8H, m, ArH), 8.1 (1H, dd, J 8, 1.5Hz, 4-H), 8.3 (1H, dd, J 4.5, 1.5Hz, 6-H), and 10.1 (1H, br s, NH).

## Claims

1. A compound of formula I, or a salt thereof: 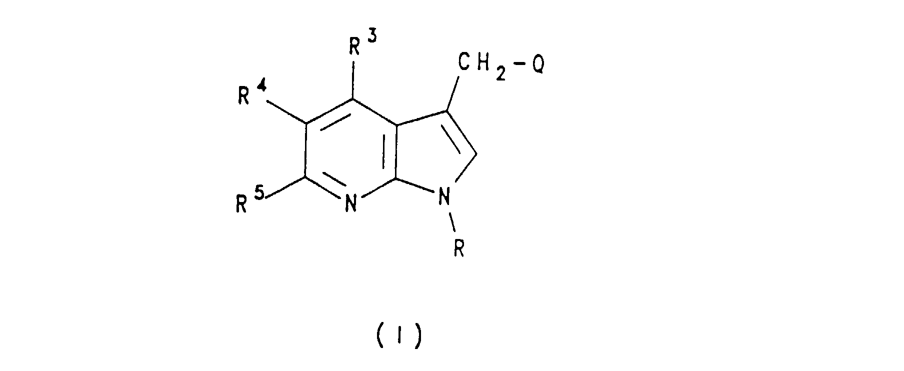 wherein
R represents hydrogen or C₁₋₆ alkyl;
Q represents a moiety of formula Qa, Qb or Qc: 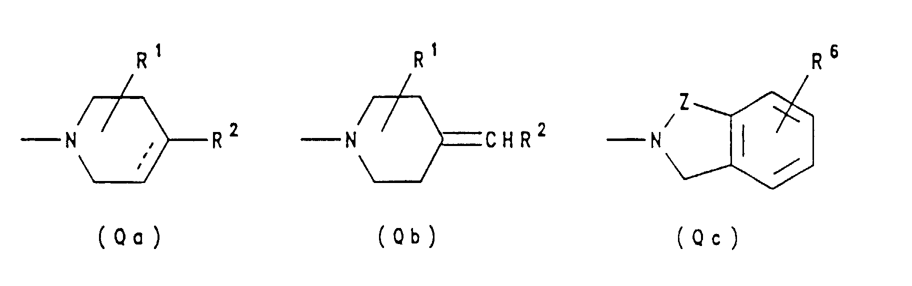 in which the broken line represents an optional chemical bond;
R¹ represents hydrogen;
R² represents C₁₋₆ alkyl, aryl, aryl(C₁₋₆)alkyl, aryloxy(C₁₋₆)alkyl, aryl(C₂₋₆)alkenyl, aryl(C₂₋₆)alkynyl, C₃₋₇ heterocycloalkyl(C₁₋₆)alkyl, heteroaryl, heteroaryl(C₁₋₆)alkyl or heteroaryl(C₂₋₆)alkenyl, any of which groups may be optionally substituted by one or more groups selected from C₁₋₆ alkyl, halogen, nitro, C₁₋₆ alkoxy, aryloxy and arylcarbonyloxy;
R³, R⁴ and R⁵ independently represent hydrogen, halogen, cyano, trifluoromethyl, nitro, amino, C₁₋₆ alkylamino, di(C₁₋₆)alkylamino, C₁₋₆ alkyl, C₁₋₆ alkoxy, aryl(C₁₋₆)alkoxy or C₂₋₆ alkylcarbonyl;
Z represents -CH₂- or -CH₂CH₂-; and
R⁶ represents hydrogen, methoxy, phenyl, chlorophenyl, phenoxy, benzyloxy, thienyl, chloro or bromo.

2. A compound as claimed in claim 1 represented by formula IIA, and salts thereof: 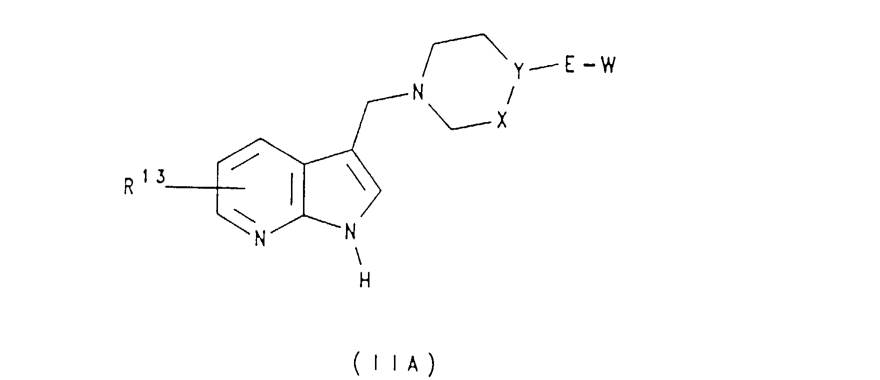 wherein
E represents -(CH₂)ₙ-, -CH=CH- or -C≡C-;
n is zero, 1, 2 or 3;
-X-Y- represents -CH₂-CH- or -CH=C-;
W represents a group of formula (i), (ii), (iii), (iv), (v) or (vi): 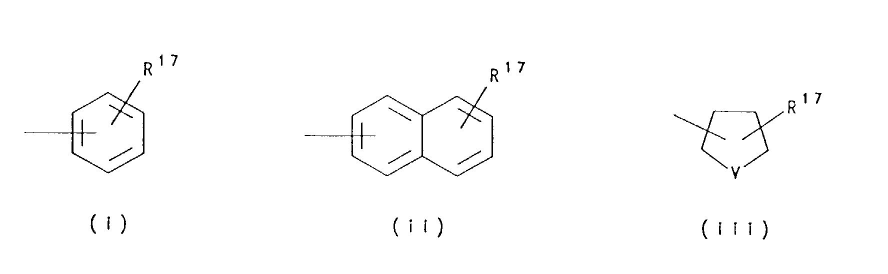 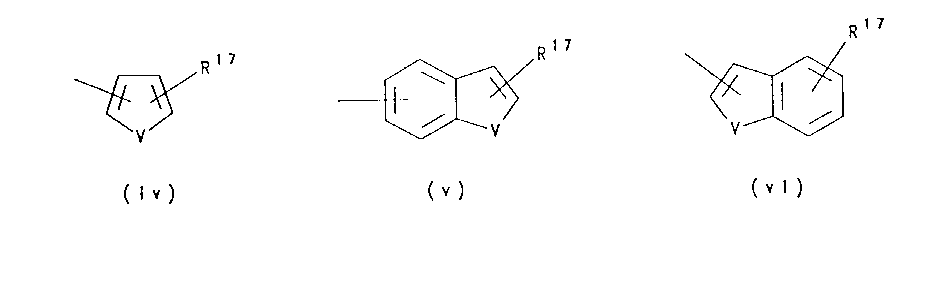 in which V represents oxygen, sulphur or NH;
R¹³ represents hydrogen, halogen, cyano, nitro, trifluoromethyl, amino, C₁₋₆ alkylamino, di(C₁₋₆)alkylamino, C₁₋₆ alkyl, C₁₋₆ alkoxy, aryl(C₁₋₆)alkoxy or C₂₋₆ alkylcarbonyl; and
R¹⁷ represents hydrogen, halogen, nitro, C₁₋₆ alkyl. or C₁₋₆ alkoxy;
provided that, when W represents a group of formula (iii), then E represents -(CH₂)ₙ- in which n is 1, 2 or 3; and also that, when W represents a group of formula (iv), (v) or (vi), then E does not represent -C≡C-.

3. A compound as claimed in claim 2 represented by formula IIB, and salts thereof: 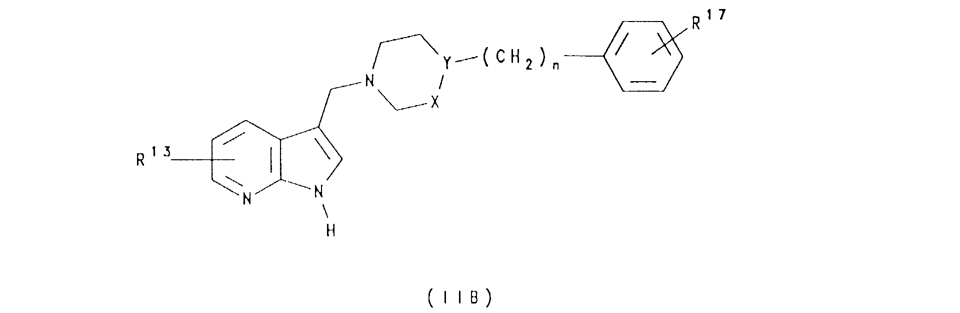 wherein n, X, Y, R¹³ and R¹⁷ are as defined in claim 2.

4. A compound as claimed in claim 1 represented by formula IIC, and salts thereof: 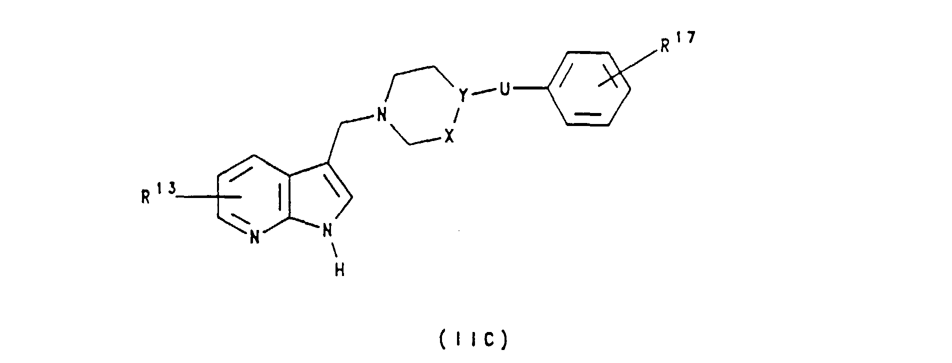 wherein
U represents -CH=CH-, -C≡C- or -CH₂O-; and
X, Y, R¹³ and R¹⁷ are as defined in claim 2.

5. A compound as claimed in claim 4 wherein U represents -CH=CH-.

6. A compound as claimed in claim 1 represented by formula IID, and salts thereof: 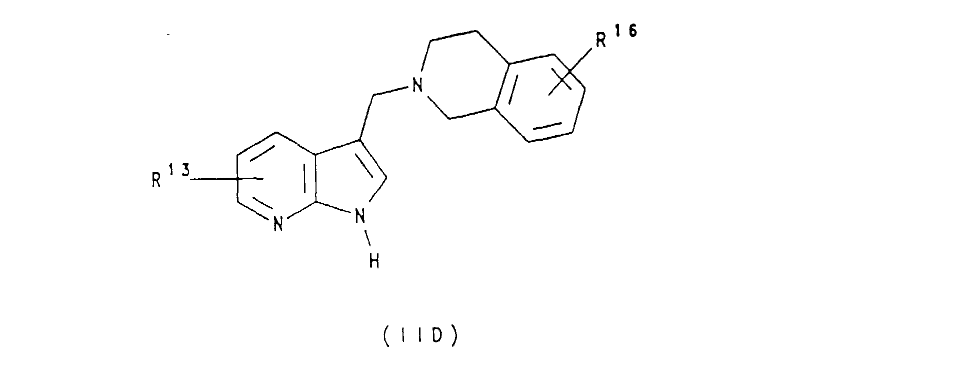 wherein
R¹³ is as defined in claim 2; and
R¹⁶ represents hydrogen, methoxy, phenyl, chlorophenyl, phenoxy, benzyloxy, thienyl, chloro or bromo.

7. A compound as claimed in claim 1 selected from:
3-(1,2,3,4-tetrahydroisoquinolin-2-yl)methyl-1H-pyrrolo[2,3-b]pyridine;
3-[4-(2-phenylethyl)piperidin-1-yl]methyl-1H-pyrrolo[2,3-b]pyridine;
3-(4-phenyl-1,2,3,6-tetrahydropyrid-1-yl)methyl-1H-pyrrolo[2,3-b]pyridine;
(E)-3-[4-(2-phenylethenyl)-1,2,3,6-tetrahydropyrid-1-yl]methyl-1H-pyrrolo[2,3-b]pyridine;
3-[4-(2-phenylethyl)-1,2,3,6-tetrahydropyrid-1-yl]methyl-1H-pyrrolo[2,3-b]pyridine;
3-[4-(naphth-2-yl)-1,2,3,6-tetrahydropyridin-1-yl]methyl-1H-pyrrolo[2,3-b]pyridine;
3-[4-(4-methoxyphenyl)-1,2,3,6-tetrahydropyridin-1-yl]methyl-1H-pyrrolo[2,3-b]pyridine;
3-[4-(1H-indol-5-yl)-1,2,3,6-tetrahydropyridin-1-yl]methyl-1H-pyrrolo[2,3-b]pyridine;
3-[4-(benzofuran-5-yl)-1,2,3,6-tetrahydropyridin-1-yl]methyl-1H-pyrrolo[2,3-b]pyridine;
3-[4-(benzofuran-6-yl)-1,2,3,6-tetrahydropyridin-1-yl]methyl-1H-pyrrolo[2,3-b]pyridine;
3-[4-(benzothiophen-2-yl)-1,2,3,6-tetrahydropyridin-1-yl]methyl-1H-pyrrolo[2,3-b]pyridine;
3-[4-(benzofuran-2-yl)-1,2,3,6-tetrahydropyridin-1-yl]methyl-1H-pyrrolo[2,3-b]pyridine;
3-[4-(2-phenylethynyl)-1,2,3,6-tetrahydropyridin-1-yl]methyl-1H-pyrrolo[2,3-b]pyridine;
(E)-3-[4-(2-(thiophen-3-yl)ethenyl)-1,2,3,6-tetrahydropyridin-1-yl]methyl-1H-pyrrolo[2,3-b]pyridine;
(E)-3-[4-(2-(2-chlorophenyl)ethenyl)-1,2,3,6-tetrahydropyridin-1-yl]methyl-1H-pyrrolo[2,3-b]pyridine;
(E)-3-[4-(2-(4-chlorophenyl)ethenyl)-1,2,3,6-tetrahydropyridin-l-yl]methyl-1H-pyrrolo[2,3-b]pyridine;
(E)-3-[4-(2-(thiophen-2-yl)ethenyl)-1,2,3,6-tetrahydropyridin-1-yl]methyl-1H-pyrrolo[2,3-b]pyridine;
3-[4-(2-(furan-2-yl)ethyl)-1,2,3,6-tetrahydropyridin-1-yl]methyl-1H-pyrrolo[2,3-b]pyridine;
3-[4-(2-(tetrahydrofuran-2-yl)ethyl)-1,2,3,6-tetrahydropyridin-l-yl]methyl-1H-pyrrolo[2,3-b]pyridine;
3-[4-(2-(5-methylfuran-2-yl)ethyl)-1,2,3,6-tetrahydropyridin-1-yl]methyl-1H-pyrrolo[2,3-b]pyridine;
3-(4-ethyl-1,2,3,6-tetrahydropyridin-1-yl)methyl-1H-pyrrolo[2,3-b]pyridine;
3-(4-benzoyloxymethyl-1,2,3,6-tetrahydropyridin-1-yl)methyl-1H-pyrrolo[2,3-b]pyridine;
(E)-3-[4-(2-(3-methoxyphenyl)ethenyl)-1,2,3,6-tetrahydropyridin-1-yl]methyl-1H-pyrrolo[2,3-b]pyridine;
3-(4-phenoxymethyl-1,2,3,6-tetrahydropyridin-1-yl)methyl-1H-pyrrolo[2,3-b]pyridine;
(E)-3-[4-(2-(5-methylfuran-2-yl)ethenyl)-1,2,3,6-tetrahydropyridin-1-yl]methyl-1H-pyrrolo[2,3-b]pyridine;
3-(6-phenyl-1,2,3,4-tetrahydroisoquinolin-2-yl)methyl-1H-pyrrolo[2,3-b]pyridine;
3-(6-methoxy-1,2,3,4-tetrahydroisoquinolin-2-yl)methyl-1H-pyrrolo[2,3-b]pyridine;
3-(7-phenyl-1,2,3,4-tetrahydroisoquinolin-2-yl)methyl-1H-pyrrolo[2,3-b]pyridine;
3-(7-benzyloxy-1,2,3,4-tetrahydroisoquinolin-2-yl)methyl-1H-pyrrolo[2,3-b]pyridine;
3-[7-(thiophen-3-yl)-1,2,3,4-tetrahydroisoquinolin-2-yl]methyl-1H-pyrrolo[2,3-b]pyridine;
3-[6-(4-chlorophenyl)-1,2,3,4-tetrahydroisoquinolin-2-yl]methyl-1H-pyrrolo[2,3-b]pyridine;
3-(5-phenyl-1,2,3,4-tetrahydroisoquinolin-2-yl)methyl-1H-pyrrolo[2,3-b]pyridine;
3-(5-benzyloxy-1,2,3,4-tetrahydroisoquinolin-2-yl)methyl-1H-pyrrolo[2,3-b]pyridine;
3-(5-phenoxy-1,2,3,4-tetrahydroisoquinolin-2-yl)methyl-1H-pyrrolo[2,3-b]pyridine;
and salts thereof.

8. A pharmaceutical composition comprising a compound of formula I as defined in claim 1 or a pharmaceutically acceptable salt thereof in association with a pharmaceutically acceptable carrier.

9. A compound of formula I as defined in claim 1 or a pharmaceutically acceptable salt thereof for use in therapy.

10. The use of a compound of formula I as defined in claim 1 or a pharmaceutically acceptable salt thereof for the manufacture of a medicament for the treatment and/or prevention of disorders of the dopamine system.

11. A process for the preparation of a compound as claimed in claim 1 which comprises:
(A) reacting a compound of formula III with a compound of formula IV: 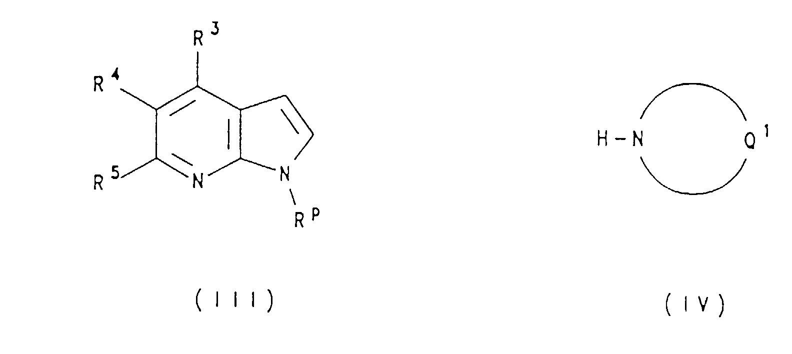 wherein R³, R⁴ and R⁵ are as defined in claim 1, Q¹ represents the residue of a moiety of formula Qa to Qc as defined in claim 1, and R^{p} corresponds to the group R as defined in claim 1 or represents a suitable protecting group; in the presence of a substantially equimolar amount of formaldehyde; followed, where required, by removal of the protecting group R^{p}; and subsequently, if necessary, N-alkylation by standard methods to introduce the moiety R; or
(B) reacting a compound of formula IV as defined above with a compound of formula V: 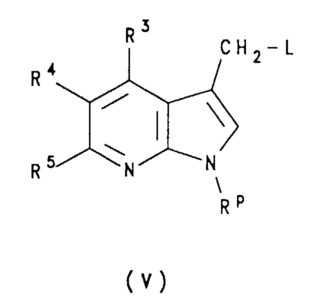 wherein R³, R⁴ and R⁵ are as defined in claim 1, R^{p} is as defined above, and L represents a suitable leaving group; followed, where required, by removal of the protecting group R^{p}; and subsequently, if necessary, N-alkylation by standard methods to introduce the moiety R; and
(C) subsequently, where required, converting a compound of formula I initially obtained into a further compound of formula I by conventional methods.

## Patentansprüche

1. Eine Verbindung der Formel I oder ein Salz davon: 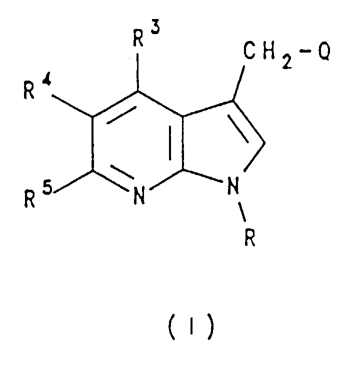 worin
R Wasserstoff oder C₁₋₆-Alkyl bedeutet,
Q einen Rest der Formel Qa, Qb oder Qc bedeutet: 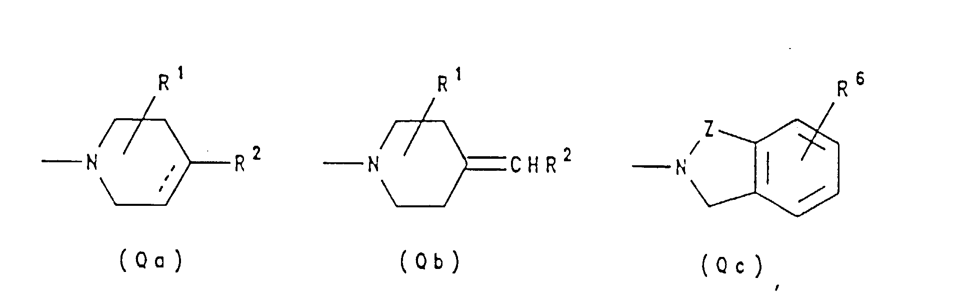 worin die gestrichelte Linie eine fakultative chemische Bindung bedeutet,
R¹ Wasserstoff bedeutet,
R² C₁₋₆-Alkyl, Aryl, Aryl(C₁₋₆)alkyl, Aryloxy(C₁₋₆)alkyl, Aryl(C₂₋₆)alkenyl, Aryl(C₂₋₆)alkinyl, C₃₋₇-Heterocycloalkyl(C₁₋₆)alkyl, Heteroaryl, Heteroaryl(C₁₋₆)alkyl oder Heteroaryl(C₂₋₆)alkenyl bedeutet, wobei jede der Gruppen gegebenenfalls durch eine oder mehrere Gruppen, ausgewählt aus C₁₋₆-Alkyl, Halogen, Nitro, C₁₋₆-Alkoxy, Aryloxy und Arylcarbonyloxy, substituiert sein kann,
R³, R⁴ und R⁵ unabhängig Wasserstoff, Halogen, Cyano, Trifluormethyl, Nitro, Amino, C₁₋₆-Alkylamino, Di(C₁₋₆)alkylamino, C₁₋₆-Alkyl, C₁₋₆-Alkoxy, Aryl(C₁₋₆)alkoxy oder C₂₋₆-Alkylcarbonyl bedeuten,
Z -CH₂- oder -CH₂CH₂- bedeutet und
R⁶ Wasserstoff, Methoxy, Phenyl, Chlorphenyl, Phenoxy, Benzyloxy, Thienyl, Chlor oder Brom bedeuten.

2. Eine wie in Anspruch 1 beanspruchte Verbindung, dargestellt durch Formel IIA, und Salze davon: 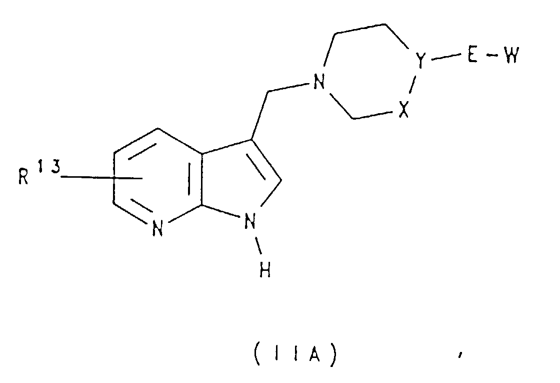 worin
E -(CH₂)ₙ-, -CH=CH- oder -C≡C- bedeutet,
n null, 1, 2 oder 3 ist,
-X-Y- -CH₂-CH- oder -CH=C- bedeutet,
W eine Gruppe der Formel (i), (ii), (iii), (iv), (v) oder (vi) bedeutet: 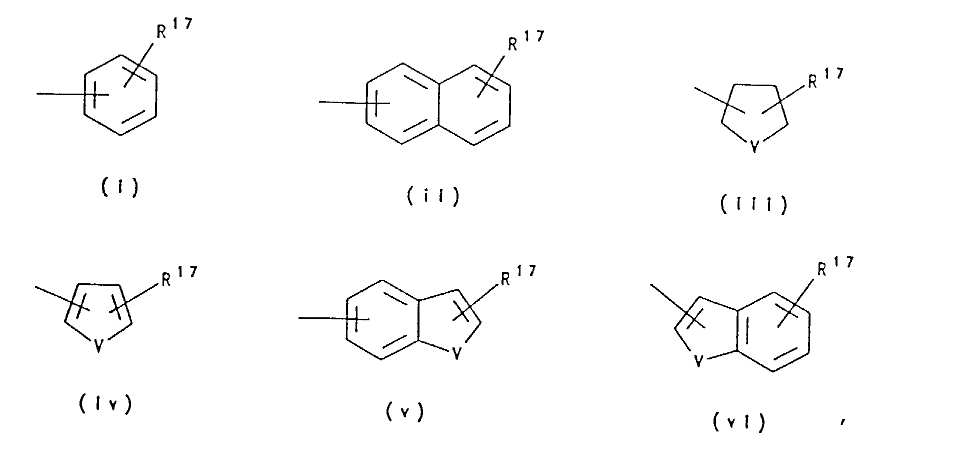 worin V Sauerstoff, Schwefel oder NH bedeutet,
R¹³ Wasserstoff, Halogen, Cyano, Nitro, Trifluormethyl, Amino, C₁₋₆-Alkylamino, Di(C₁₋₆)alkylamino, C₁₋₆-Alkyl, C₁₋₆-Alkoxy, Aryl(C₁₋₆)-alkoxy oder C₂₋₆-Alkylcarbonyl bedeutet und
R¹⁷ Wasserstoff, Halogen, Nitro, C₁₋₆-Alkyl oder C₁₋₆-Alkoxy bedeutet,
mit der Maßgabe, daß, wenn W eine Gruppe der Formel (iii) ist, E dann -(CH₂)ₙ- bedeutet, worin n 1, 2 oder 3 ist, und auch, daß, wenn W eine Gruppe der Formel (iv), (v) oder (vi) bedeutet, E dann nicht -C≡C- bedeutet.

3. Eine wie in Anspruch 2 beanspruchte Verbindung, dargestellt durch Formel IIB, und Salze davon: 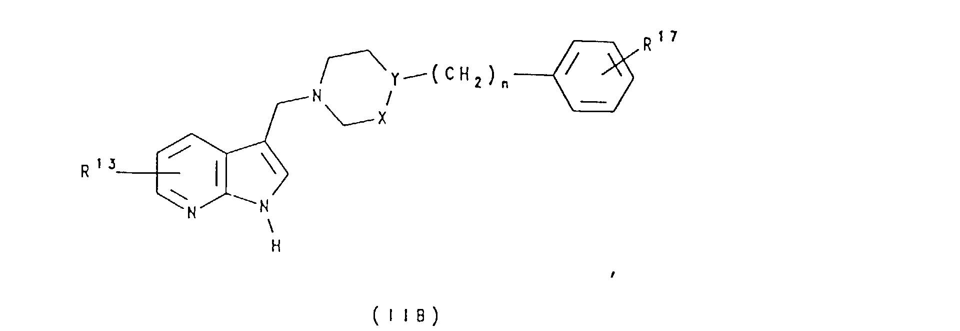 worin n, X, Y, R¹³ und R¹⁷ wie in Anspruch 2 definiert sind.

4. Eine wie in Anspruch 1 beanspruchte Verbindung, dargestellt durch Formel IIC, und Salze davon: 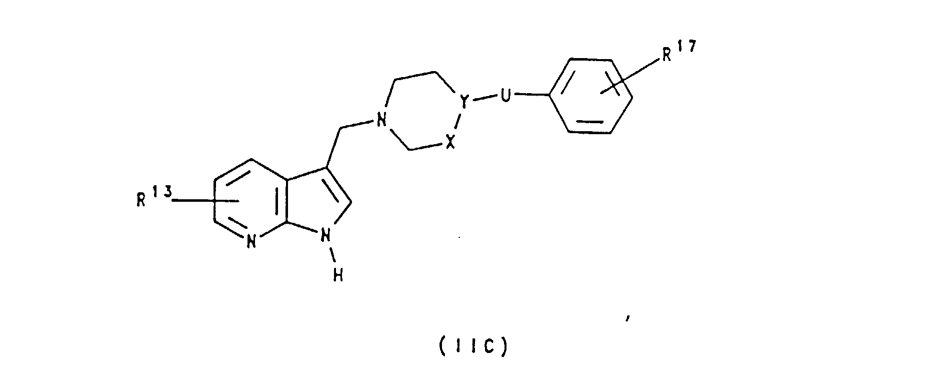 worin
U -CH=CH-, -C≡C- oder -CH₂O- bedeutet und
X, Y, R¹³ und R¹⁷ wie in Anspruch 2 definiert sind.

5. Eine wie in Anspruch 4 beanspruchte Verbindung, worin U -CH=CH- bedeutet.

6. Eine wie in Anspruch 1 beanspruchte Verbindung, dargestellt durch Formel IID, und Salze davon: 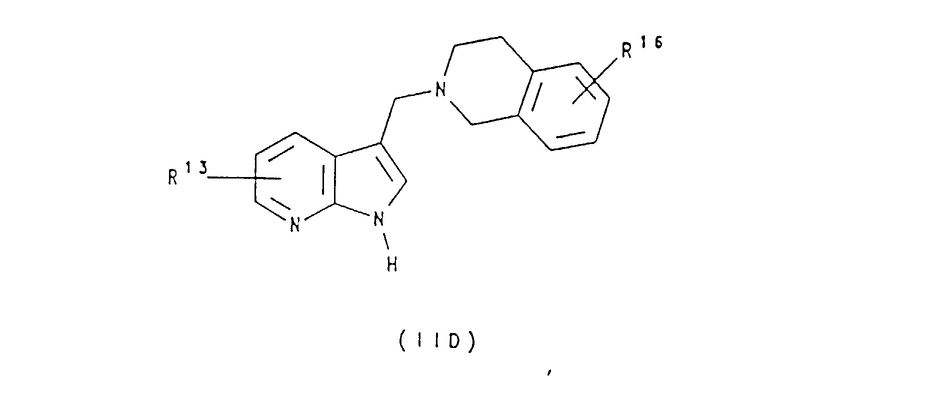 worin
R¹³ wie in Anspruch 2 definiert ist und
R¹⁶ Wasserstoff, Methoxy, Phenyl, Chlorphenyl, Phenoxy, Benzyloxy, Thienyl, Chlor oder Brom bedeutet.

7. Eine wie in Anspruch 1 beanspruchte Verbindung, ausgewählt aus:
3-(1,2,3,4-Tetrahydroisochinolin-2-yl)methyl-1H-pyrrolo[2,3-b]pyridin,
3-[4-(2-Phenylethyl)piperidin-1-yl]methyl-1H-pyrrolo[2,3-b]pyridin,
3-(4-Phenyl-1,2,3,6-tetrahydropyrid-1-yl)methyl-1H-pyrrolo[2,3-b]pyridin,
(E)-3-[4-(2-Phenylethenyl)-1,2,3,6-tetrahydropyrid-1-yl]methyl-1H-pyrrolo[2,3-b]pyridin,
3-[4-(2-Phenylethyl)-1,2,3,6-tetrahydropyrid-1-yl]methyl-1H-pyrrolo[2,3-b]pyridin,
3-[4-(Naphth-2-yl)-1,2,3,6-tetrahydropyridin-1-yl]methyl-1H-pyrrolo[2,3-b]pyridin,
3-[4-(4-Methoxyphenyl)-1,2,3,6-tetrahydropyridin-1-yl]methyl-1H-pyrrol[2,3-b]pyridin,
3-[4-(1H-Indol-5-yl)-1,2,3,6-tetrahydropyridin-1-yl]methyl-1H-pyrrolo[2,3-b]pyridin,
3-[4-(Benzofuran-5-yl)-1,2,3,6-tetrahydropyridin-1-yl]methyl-1H-pyrrolo[2,3-b]pyridin,
3-[4-(Benzofuran-6-yl)-1,2,3,6-tetrahydropyridin-1-yl]methyl-1H-pyrrolo[2,3-b]pyridin,
3-[4-(Benzothiophen-2-yl)-1,2,3,6-tetrahydropyridin-1-yl]methyl-1H-pyrrolo[2,3-b]pyridin,
3-[4-(Benzofuran-2-yl)-1,2,3,6-tetrahydropyridin-1-yl]methyl-1H-pyrrolo[2,3-b]pyridin,
3-[4-(2-Phenylethinyl)-1,2,3,6-tetrahydropyridin-1-yl]methyl-1H-pyrrolo[2,3-b]pyridin,
(E)-3-[4-(2-(Thiophen-3-yl)ethenyl)-1,2,3,6-tetrahydropyridin-1-yl]methyl-1H-pyrrolo[2,3-b]pyridin,
(E)-3-[4-(2-(2-Chlorphenyl)ethenyl)-1,2,3,6-tetrahydropyridin-1-yl]methyl-1H-pyrrolo[2,3-b]pyridin,
(E)-3-[4-(2-(4-Chlorphenyl)ethenyl)-1,2,3,6-tetrahydropyridin-1-yl]methyl-1H-pyrrolo[2,3-b]pyridin,
(E)-3-[4-(2-(Thiophen-2-yl)ethenyl)-1,2,3,6-tetrahydropyridin-1-yl]methyl-1H-pyrrolo[2,3-b]pyridin,
3-[4-(2-(Furan-2-yl)ethyl)-1,2,3,6-tetrahydropyridin-1-yl]methyl-1H-pyrrolo[2,3-b]pyridin,
3-[4-(2-(Tetrahydrofuran-2-yl)ethyl)-1,2,3,6-tetrahydropyridin-1-yl]methyl-1H-pyrrolo[2,3-b]pyridin,
3-[4-(2-(5-Methylfuran-2-yl)ethyl)-1,2,3,6-tetrahydropyridin-1-yl]methyl-1H-pyrrolo[2,3-b]pyridin,
3-(4-Ethyl-1,2,3,6-tetrahydropyridin-1-yl)methyl-1H-pyrrolo[2,3-b]pyridin,
3-(4-Benzoyloxymethyl-1,2,3,6-tetrahydropyridin-1-yl)methyl-1H-pyrrolo[2,3-b]pyridin,
(E)-3-[4-(2-(3-Methoxyphenyl)ethenyl)-1,2,3,6-tetrahydropyridin-1-yl]methyl-1H-pyrrolo[2,3-b]pyridin,
3-(4-Phenoxymethyl-1,2,3,6-tetrahydropyridin-1-yl)methyl-1H-pyrrolo[2,3-b]pyridin,
(E)-3-[4-(2-(5-Methylfuran-2-yl)ethenyl)-1,2,3,6-tetrahydropyridin-1-yl]methyl-1H-pyrrolo[2,3-b]pyridin,
3-(6-Phenyl-1,2,3,4-tetrahydroisochinolin-2-yl)methyl-1H-pyrrolo[2,3-b]pyridin,
3-(6-Methoxy-1,2,3,4-tetrahydroisochinolin-2-yl)methyl-1H-pyrrolo[2,3-b]pyridin,
3-(7-Phenyl-1,2,3,4-tetrahydroisochinolin-2-yl)methyl-1H-pyrrolo[2,3-b]pyridin,
3-(7-Benzyloxy-1,2,3,4-tetrahydroisochinolin-2-yl)methyl-1H-pyrrolo[2,3-b]pyridin,
3-[7-(Thiophen-3-yl)-1,2,3,4-tetrahydroisochinolin-2-yl]methyl-1H-pyrrolo[2,3-b]pyridin,
3-[6-(4-Chlorphenyl)-1,2,3,4-tetrahydroisochinolin-2-yl]methyl-1H-pyrrolo[2,3-b]pyridin,
3-(5-Phenyl-1,2,3,4-tetrahydroisochinolin-2-yl)methyl-1H-pyrrolo[2,3-b]pyridin,
3-(5-Benzyloxy-1,2,3,4-tetrahydroisochinolin-2-yl)methyl-1H-pyrrolo[2,3-b]pyridin,
3-(5-Phenoxy-1,2,3,4-tetrahydroisochinolin-2-yl)methyl-1H-pyrrolo[2,3-b]pyridin,
und Salze davon.

8. Eine pharmazeutische Zusammensetzung, die eine wie in Anspruch 1 definierte Verbindung der Formel I oder ein pharmazeutisch annehmbares Salz davon in Verbindung mit einem pharmazeutisch annehmbaren Träger enthält.

9. Eine wie in Anspruch 1 definierte Verbindung der Formel I oder ein pharmazeutisch annehmbares Salz davon zur Verwendung in der Therapie.

10. Die Verwendung einer wie in Anspruch 1 definierten Verbindung der Formel I oder eines pharmazeutisch annehmbaren Salzes davon zur Herstellung eines Medikaments zur Behandlung und/oder Prävention von Störungen des Dopaminsystems.

11. Ein Verfahren zur Herstellung einer wie in Anspruch 1 beanspruchten Verbindung, das umfaßt:
(A) Umsetzung einer Verbindung der Formel III mit einer Verbindung der Formel IV: 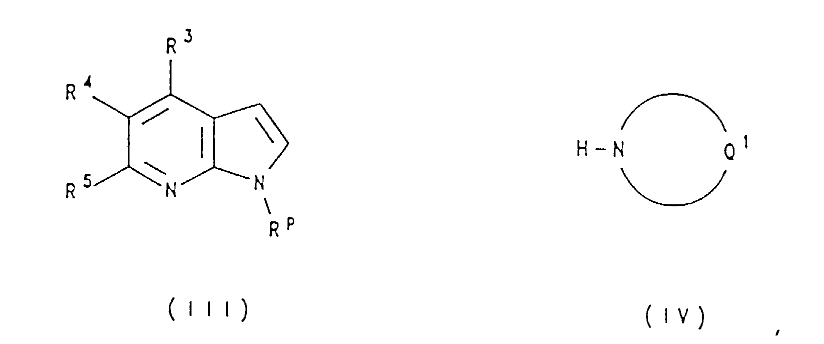 worin R³, R⁴ und R⁵ wie in Anspruch 1 definiert sind, Q¹ den Rest einer wie in Anspruch 1 definierten Gruppe der Formel Qa bis Qc bedeutet, und R^{p} der wie in Anspruch 1 definierten Gruppe R entspricht oder eine geeignete Schutzgruppe bedeutet, in Gegenwart einer im wesentlichen äquimolaren Menge Formaldehyd, gefolgt, wo erforderlich, von der Entfernung der Schutzgruppe R^{p} und anschließend, falls notwendig, von der N-Alkylierung durch Standardverfahren, um den Rest R einzuführen, oder
(B) Umsetzung einer wie oben definierten Verbindung der Formel IV mit einer Verbindung der Formel V:

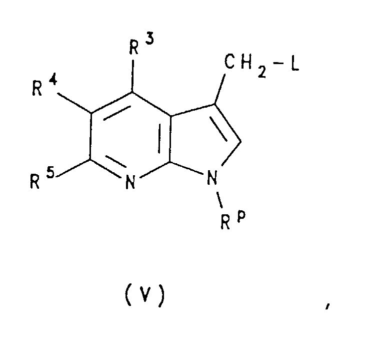

## Revendications

1. Composé de formule I, ou un sel de celui-ci: 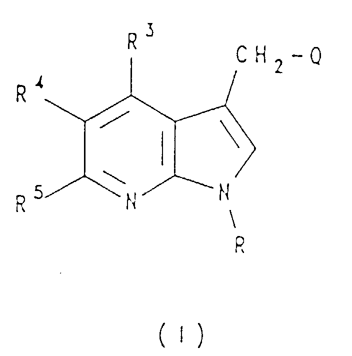 dans laquelle
R représente un atome d'hydrogène ou un groupe alkyle en C₁-C₆;
Q représente un groupement de formule Qa, Qb ou Qc: 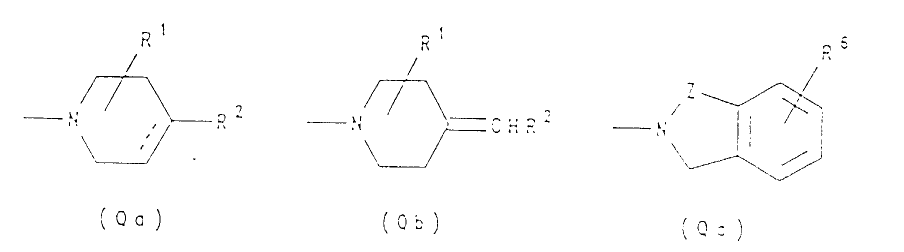 dans laquelle la ligne pointillée représente une liaison chimique facultative;
R¹ représente un atome d'hydrogène;
R² représente un groupe alkyle en C₁-C₆, aryle, aryl(alkyle en C₁-C₆), aryloxy(alkyle en C₁-C₆), aryl(alcényle en C₂-C₆); aryl(alcynyle en C₂-C₆), (hétérocycloalkyl en C₃-C₇)(alkyle en C₁-C₆), hétéroaryle, hétéroaryl(alkyle en C₁-C₆), ou hétéroaryl(alcényle en C₂-C₆), l'un quelconque de ces groupes pouvant être éventuellement substitué par un ou plusieurs groupes choisis parmi les groupes alkyle en C₁-C₆, halogéno, nitro, alcoxy en C₁-C₆, aryloxy et arylcarbonyloxy;
R³, R⁴ et R⁵ représentent chacun indépendamment un atome d'hydrogène, un atome d'halogène, un groupe cyano, trifluorométhyle, nitro, amino, (alkyl en C₁-C₆)amino, di(alkyl en C₁-C₆)amino, alkyle en C₁-C₆, alcoxy en C₁-C₆, arylalcoxy en C₁-C₆ ou (alkyl en C₂-C₆)carbonyle;
Z représente -CH₂- ou -CH₂CH₂-; et
R⁶ représente un atome d'hydrogène, un groupe méthoxy, phényle, chlorophényle, phénoxy, benzyloxy, thiényle, chloro ou bromo.

2. Composé selon la revendication 1 représenté par la formule IIA, et les sels de celui-ci: 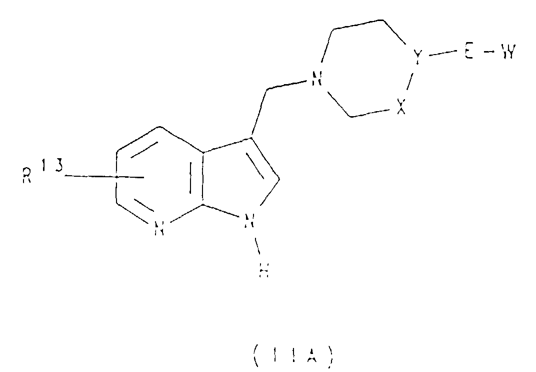 dans laquelle
E représente -(CH₂)ₙ-, -CH=CH- ou -C≡C-;
n vaut zéro, 1, 2 ou 3;
-X-Y- représente -CH₂CH- ou -CH=C-;
W représente un groupe de formule (i), (ii), (iii), (iv), (v) ou (vi): 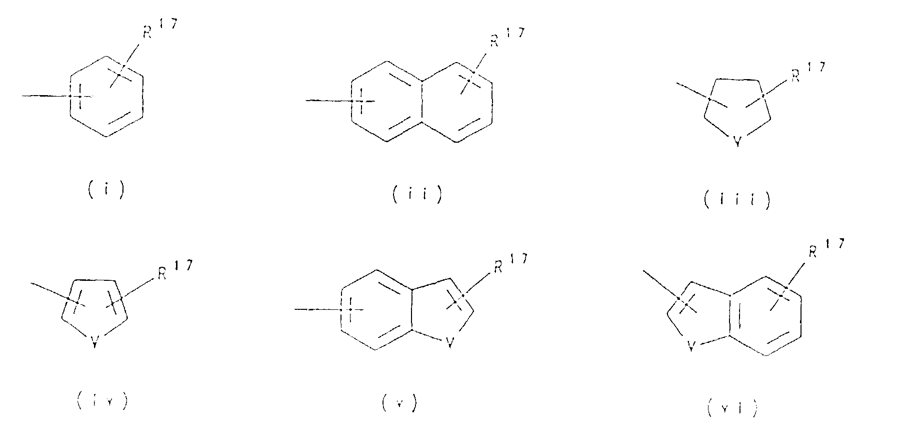 dans laquelle V représente un atome d'oxygène, de soufre ou NH;
R¹³ représente un atome d'hydrogène, un atome d'halogène, un groupe cyano, nitro, trifluorométhyle, amino, (alkyl en C₁-C₆)amino, di(alkyl en C₁-C₆)amino, alkyle en C₁-C₆, alcoxy en C₁-C₆, aryl(alcoxy en C₁-C₆) ou (alkyl en C₂-C₆)carbonyle; et
R¹⁷ représente un atome d'hydrogène, un atome d'halogène, un groupe nitro, alkyle en C₁-C₆ ou alcoxy en C₁-C₆;
à condition que, lorsque W représente un groupe de formule (iii), alors E représente -(CH₂)ₙ- où n vaut 1, 2 ou 3, et également lorsque W représente un groupe de formule (iv), (v) ou (vi), alors E ne représente pas -C≡C-.

3. Composé selon la revendication 2 représenté par la formule IIA, et les sels de celui-ci: 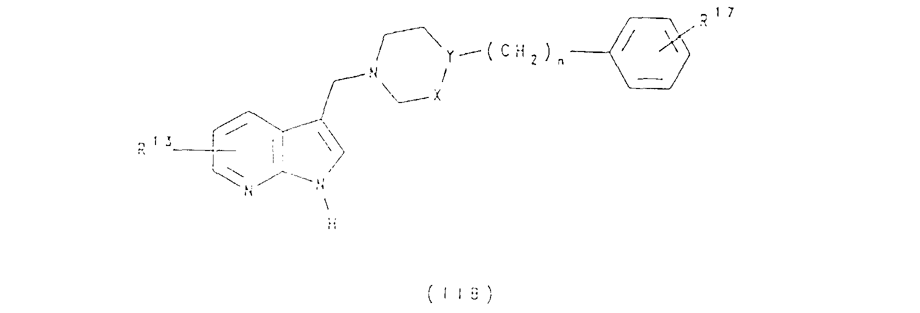 dans laquelle n, X, Y, R¹³ et R¹⁷ sont tels que définis dans la revendication 2.

4. Composé selon la revendication 1 représenté par la formule IIC, et les sels de celui-ci: 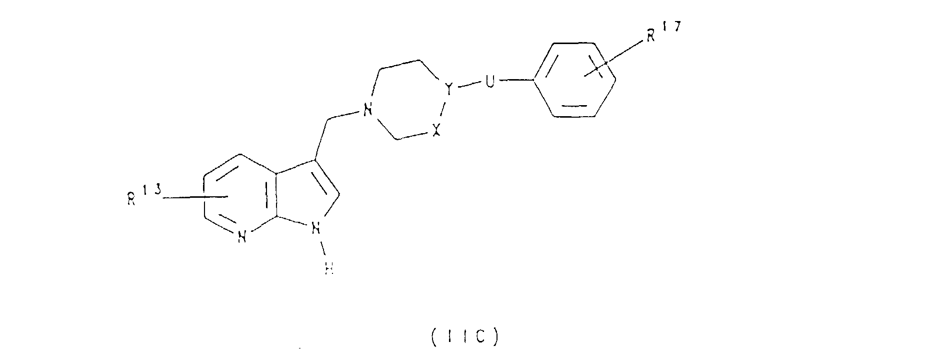 dans laquelle
U représente -CH=CH-, -C≡C- ou -CH₂O-; et
X, Y, R¹³ et R¹⁷ sont tels que définis dans la revendication 2.

5. Composé selon la revendication 4 dans lequel U représente -CH=CH-.

6. Composé selon la revendication 1 représenté par la formule IID et les sels de celui-ci: 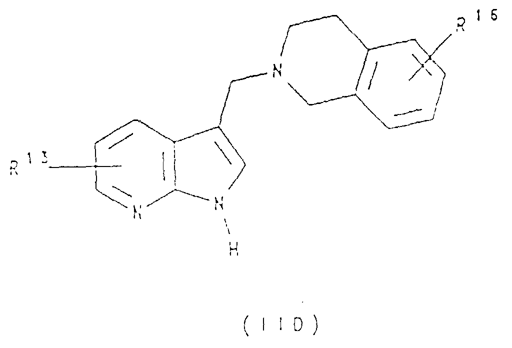 dans laquelle
R¹³ est tel que défini dans la revendication 2; et
R¹⁶ représente un atome d'hydrogène, un groupe méthoxy, phényle, chlorophényle, phénoxy, benzyloxy, thiényle, chloro ou bromo.

7. Composé selon la revendication 1 choisi parmi:
la 3-(1,2,3,4-tétrahydroisoquinoléin-2-yl)méthyl-1H-pyrrolo[2,3-b]-pyridine;
la 3-[4-(2-phényléthyl)pipéridin-1-yl]méthyl-1H-pyrrolo[2,3-b]-pyridine;
la 3-(4-phényl-1,2,3,6-tétrahydropyrid-1-yl)méthyl-1H-pyrrolo-[2,3-b]pyridine;
la (E)-3-[4-(2-phényléthényl)-1,2,3,6-tétrahydropyrid-1-yl]méthyl-1H-pyrrolo[2,3-b]pyridine;
la 3-[4-(2-phényléthyl)-1,2,3,6-tétrahydropyrid-1-yl]méthyl-1H-pyrrolo[2,3-b]pyridine;
la 3-[4-(napht-2-yl)-1,2,3,6-tétrahydropyridin-1-yl]méthyl-1H-pyrrolo[2,3-b]pyridine;
la 3-[4-(4-méthoxyphényl)-1,2,3,6-tétrahydropyridin-1-yl]méthyl-1H-pyrrolo[2,3-b]pyridine;
la 3-[4-(1H-indol-5-yl)-1,2,3,6-tétrahydropyridin-1-yl]méthyl-1H-pyrrolo[2,3-b]pyridine;
la 3-[4-(benzofuran-5-yl)-1,2,3,6-tétrahydropyridin-1-yl]méthyl-1H-pyrrolo[2,3-b]pyridine;
la 3-[4-(benzofuran-6-yl)-1,2,3,6-tétrahydropyridin-1-yl]méthyl-1H-pyrrolo[2,3-b]pyridine;
la 3-[4-(benzothiophén-2-yl)-1,2,3,6-tétrahydropyridin-1-yl]méthyl-1H-pyrrolo[2,3-b]pyridine;
la 3-[4-(benzofuran-2-yl)-1,2,3,6-tétrahydropyridin-1-yl]méthyl-1H-pyrrolo[2,3-b]pyridine;
la 3-[4-(2-phényléthynyl)-1,2,3,6-tétrahydropyridin-1-yl]méthyl-1H-pyrrolo[2,3-b]pyridine;
la (E)-3-[4-(2-(thiophén-3-yl)éthényl)-1,2,3,6-tétrahydropyridin-1-yl]méthyl-1H-pyrrolo[2,3-b]pyridine;
la (E)-3-[4-(2-(2-chlorophényl)éthényl)-1,2,3,6-tétrahydropyridin-1-yl]méthyl-1H-pyrrolo[2,3-b]pyridine;
la (E)-3-[4-(2-(4-chlorophényl)éthényl)-1,2,3,6-tétrahydropyridin-1-yl]méthyl-1H-pyrrolo[2,3-b]pyridine;
la (E)-3-[4-(2-(thiophén-2-yl)éthényl)-1,2,3,6-tétrahydropyridin-1-yl]méthyl-1H-pyrrolo[2,3-b]pyridine;
la 3-[4-(2-(furan-2-yl)éthyl)-1,2,3,6-tétrahydropyridin-1-yl]méthyl-1H-pyrrolo[2,3-b]pyridine;
la 3-[4-(2-(tétrahydrofuran-2-yl)éthyl-1,2,3,6-tétrahydropyridin-1-yl]-méthyl-1H-pyrrolo[2,3-b]pyridine;
la 3-[4-(2-(5-méthylfuran-2-yl)éthyl)-1,2,3,6-tétrahydropyridin-1-yl]-méthyl-1H-pyrrolo[2,3-b]pyridine;
la 3-(4-éthyl-1,2,3,6-tétrahydropyridin-1-yl)méthyl-1H-pyrrolo[2,3-b]pyridine;
la 3-(4-benzoyloxyméthyl-1,2,3,6-tétrahydropyridin-1-yl)méthyl-1H-pyrrolo[2,3-b]pyridine;
la (E)-3-[4-(2-(3-méthoxyphényl)éthényl)-1,2,3,6-tétrahydropyridin-1-yl]méthyl-1H-pyrrolo[2,3-b]pyridine;
la 3-(4-phénoxyméthyl-1,2,3,6-tétrahydropyridin-1-yl)méthyl-1H-pyrrolo[2,3-b]pyridine;
la (E)-3-[4-(2-(5-méthylfuran-2-yl)éthényl)-1,2,3,6-tétrahydropyridin-1-yl]méthyl-1H-pyrrolo[2,3-b]pyridine;
la 3-(6-phényl-1,2,3,4-tétrahydroisoquinoléin-2-yl)méthyl-1H-pyrrolo[2,3-b]pyridine;
la 3-(6-méthoxy-1,2,3,4-tétrahydroisoquinoléin-2-yl)méthyl-1H-pyrrolo[2,3-b]pyridine;
la 3-(7-phényl-1,2,3,4-tétrahydroisoquinoléin-2-yl)méthyl-1H-pyrrolo[2,3-b]pyridine;
la 3-(7-benzyloxy-1,2,3,4-tétrahydroisoquinoléin-2-yl)méthyl-1H-pyrrolo[2,3-b]pyridine;
la 3-[7-(thiophén-3-yl)-1,2,3,4-tétrahydroisoquinoléin-2-yl]méthyl-1H-pyrrolo[2,3-b]pyridine;
la 3-[6-(4-chlorophényl)-1,2,3,4-tétrahydroisoquinoléin-2-yl]méthyl-1H-pyrrolo[2,3-b]pyridine;
la 3-(5-phényl-1,2,3,4-tétrahydroisoquinoléin-2-yl)méthyl-1H-pyrrolo[2,3-b]pyridine;
la 3-(5-benzyloxy-1,2,3,4-tétrahydroisoquinoléin-2-yl)méthyl-1H-pyrrolo[2,3-b]pyridine;
la 3-(5-phénoxy-1,2,3,4-tétrahydroisoquinoléin-2-yl)méthyl-1H-pyrrolo[2,3-b]pyridine;
et leurs sels.

8. Composition pharmaceutique comprenant un composé de formule I tel que défini dans la revendication 1 ou un sel pharmaceutiquement acceptable de celui-ci en association avec un véhicule pharmaceutiquement acceptable.

9. Composé de formule I tel que défini dans la revendication 1 ou un sel pharmaceutiquement acceptable de celui-ci à utiliser en thérapeutique.

10. Utilisation d'un composé de formule I tel que défini dans la revendication 1 ou d'un sel pharmaceutiquement acceptable de celui-ci pour la fabrication d'un médicament pour le traitement et/ou la prévention de troubles du système dopaminergique.

11. Procédé de préparation d'un composé selon la revendication 1 qui comprend les étapes consistant à:
(A) faire réagir un composé de formule III avec un composé de formule IV: 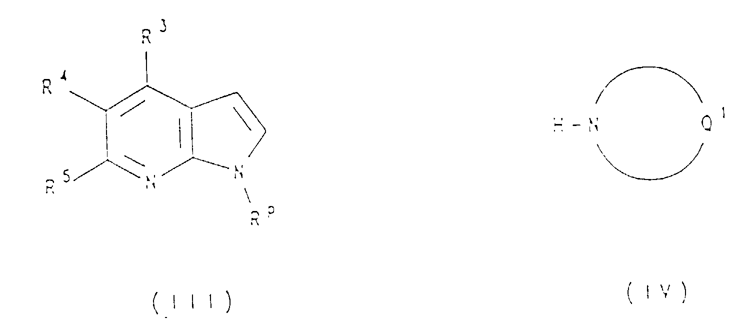 dans lesquelles R³, R⁴ et R⁵ sont tels que définis dans la revendication 1, Q¹ représente le résidu d'un groupement de formule Qa à Qc tel que défini dans la revendication 1, et R^{p} correspond au groupe R tel que défini dans la revendication 1 ou représente un groupe protecteur convenable; en présence d'une quantité essentiellement équimolaire de formaldéhyde; en continuant, le cas échéant, par l'élimination du groupe protecteur R^{p}; et ensuite, le cas échéant, par une N-alkylation par des procédés standard pour introduire le groupement R; ou
(B) faire réagir un composé de formule IV tel que défini ci-dessus avec un composé de formule V: 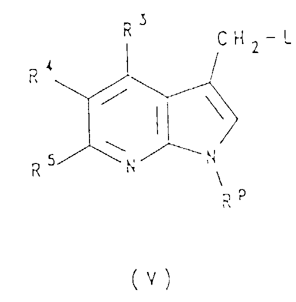 dans laquelle R³, R⁴ et R⁵ sont tels que définis dans la revendication 1, R^{p} est tel que défini ci-dessus, et L représente un groupe partant convenable; en continuant, le cas échéant, par l'élimination du groupe protecteur R^{p}; et ensuite, le cas échéant, par une N-alkylation par des procédés standard pour introduire le groupement R; et
(C) ensuite, le cas échéant, convertir un composé de formule I obtenu initialement en un autre composé de formule I par des procédés classiques.
